# EUROPEAN PATENT SPECIFICATION

(11) **EP 3 778 673 B1**
(45) Date of publication and mention of the grant of the patent: **02.11.2022**
(21) Application number: 19775463.3
(22) Date of filing: 28.03.2019
(51) Int. Cl.: C08F 220/12, A61K 8/06, A61K 8/898, A61Q 1/02, A61Q 1/04, A61Q 1/06, A61Q 1/10, A61Q 1/12, A61Q 1/14, A61Q 5/06, A61Q 17/04, A61Q 19/00, A61Q 19/02, A61Q 19/10, C08F 220/26, C08F 220/36, C08F 230/02, C08F 230/08, C08F 290/06, C08F 220/18, A61K 8/19

(54) **PHOSPHORYLCHOLINE GROUP AND SILICONE GROUP-CONTAINING COPOLYMER, POWDER COATED WITH SAID COPOLYMER AND PRODUCTION METHOD THEREFOR, AND USE OF SAID COPOLYMER AND SAID POWDER IN COSMETICS**
PHOSPHORYLCHOLINGRUPPEN- UND SILIKONGRUPPEHALTIGES COPOLYMER, MIT BESAGTEM COPOLYMER BESCHICHTETES PULVER UND HERSTELLUNGSVERFAHREN DAFÜR SOWIE VERWENDUNG DES BESAGTEN COPOLYMERS UND DES BESAGTEN PULVERS IN DER KOSMETIK
COPOLYMÈRE CONTENANT UN GROUPE DE PHOSPHORYLCHOLINE ET UN GROUPE DE SILICONE, POUDRE REVÊTUE DUDIT COPOLYMÈRE ET PROCÉDÉ DE PRODUCTION ASSOCIÉ ET UTILISATION DUDIT COPOLYMÈRE ET DE LADITE POUDRE EN COSMÉTIQUE

(30) Priority: 30.03.2018 JP 2018067406; 30.03.2018 JP 2018067804
(43) Date of publication of application: 17.02.2021
(73) Proprietor: Kose Corporation, Tokyo 103-8251 (JP); NOF Corporation, Shibuya-ku Tokyo 150-6019 (JP)
(72) Inventor: MASUBUCHI, Yuji, Tokyo 114-0005 (JP); SASAKI, Ryo, Tokyo 114-0005 (JP); TAKESHITA, Takashi, Tokyo 114-0005 (JP); MUNEKATA, Yuki, Amagasaki, Hyogo 660-0095 (JP); HARATA, Eiji, Amagasaki, Hyogo 660-0095 (JP); MARUYAMA, Kei-ichi, Amagasaki, Hyogo 660-0095 (JP)
(74) Representative: D Young & Co LLP
(86) International application number: PCT/JP2019/013675
(87) International publication number: WO 2019/189608

(56) References cited:
- WO-A1-2005/094758
- WO-A1-2017/043500
- JP-A- 2006 052 400
- JP-A- 2013 139 400
- JP-A- 2017 206 441
- JP-B2- 4 786 395
- JP-B2- 5 972 098
- US-A1- 2007 196 291
- OISHI, TSUTOMU et al.: "Synthesis of Terpolymers Having a Phospholipid Polar Group and Poly (oxyethylene) in the Side Chain and Their Protein Adsorption-Resistance Properties", Journal of Applied Polymer Science, vol. 86, 20 August 2002 (2002-08-20), pages 1092-1105, XP055638834,

## Description

### Technical Field

The present invention relates to a novel phosphorylcholine group and silicone group-containing copolymer (hereinafter, may be referred to as "MPC-SiMA polymer") having excellent properties as a raw material, in particular, for cosmetics, a powder coated with the copolymer and a method for manufacturing the same, and use of the copolymer and the powder in cosmetics.

### Background Art

It is desirable for cosmetics to satisfy both a good touch feeling and a high function. In order to acquire a good touch feeling, it is necessary to satisfy properties, such as being light, silky, smooth, moist, adhesive, slippery, and soft. On the other hand, in order to achieve a high function, it is necessary to satisfy properties, such as being strong against sweat and water, being strong against sebum, evenly adhering to skin, not causing wrinkles, having excellent touch-proof properties that makeup does not easily adhere to clothes or the like, and having high dispersibility. Since contact between skin and a powder influences these properties, it is important to control the powder surface conditions. Accordingly, various products by functionalization using surface treatment technology have been proposed so far. Incidentally, the term "touch-proof" or "touch-proof properties" means an effect that a cosmetic film does not easily come off even if a finger, handkerchief, or the like touches the cosmetic film.

As a material having excellent moisture retention, skin adhesion, safety, etc., Lipidure (registered trademark of NOF Corporation), which is a methacryloyloxyethyl phosphorylcholine (hereinafter, abbreviated to "MPC") polymer including MPC as a repeating unit, is known. MPC having a phosphorylcholine group has excellent biocompatibility (bioaffinity) and moisture retention and is used as a biocompatible material, for example, as an artificial organ, a biological membrane, a coating agent for medical equipment, a drug delivery agent, or a cosmetic ingredient. Incidentally, silicone, which has properties that are essential to cosmetic raw materials, i.e., being physiologically inactive, very safe, and stable, has been used for various purposes in the cosmetic field in both solid and liquid (silicone oil) states. For example, a copolymer including, as a repeating unit, silicone methacrylate (SiMA) having a main chain of an acrylic polymer and a modification group of a silicone portion is known to be excellent in water resistance, oil resistance, abrasion resistance, and makeup durability.

As cosmetics containing a polymer having properties of both a polymer including a phosphorylcholine group and silicone, for example, cosmetics containing a polymer including an organopolysiloxane residue and a phosphorylcholine group have been proposed (Patent Documents 1 to 3).

In addition, Patent Document 1 also discloses a polymer further including an unsaturated carboxylic acid and a derivative monomer thereof (for example, cyclohexyl (meth)acrylate or polyoxyalkylene mono(meth)acrylate) as a repeating unit in addition to the organopolysiloxane residue and the phosphorylcholine group. Further, for example, Patent Documents 4 to 6 describe the use of cyclohexyl (meth)acrylate as one component of a copolymer that is used for manufacturing cosmetics. For example, Patent Documents 7 to 10 describe the use of polyoxyalkylene mono(meth)acrylate as one component of a copolymer that is used for manufacturing cosmetics.

On the other hand, in the cosmetic field, in order to improve the dispersibility of a powder or to control the touch feeling, powders surface-treated using various surface treatment agents have been studied in the past. For example, as a powder having appropriate slipperiness and a soft touch feeling and being excellent in moist feel and adhesion to skin, a surface-treated powder prepared by coating treatment of a powder with a silicone elastomer, which is selected from crosslinked type polyether-modified silicone and crosslinked type polyglycerin-modified silicone, dispersed in a dispersant including water has been proposed (Patent Document 11).

In addition, for example, an amino-modified silicone-treated powder excellent in extensibility, adhesion, a touch feeling, punch moldability, and water repellency (Patent Document 12), a surface-treated powder with triethoxycaprylylsilane or methyl hydrogen polysiloxane with the intention of improving the dispersibility of the powder (paragraph [0021] of Patent Document 13), and a surface-treated powder prepared by treating the surface of a pigment powder with specific pseudo-ceramide represented by hydroxypropyl bispalmitamide MEA (Patent Document 14) have been proposed so far.

It is desirable for powders that are used in cosmetics to satisfy both a good touch feeling and a high function. In order to acquire a good touch feeling, powders are required to satisfy properties, such as being light, silky, smooth, moist, adhesive, slippery, and soft. On the other hand, a high function means properties, such as being strong against sweat and water, being strong against sebum, evenly adhering to skin, not causing wrinkles, having excellent touch-proof properties that makeup does not easily come off even if fingers or clothes touch it, and having high dispersibility. Since the contact between skin and a powder highly contributes to these properties, it is important to control the powder surface conditions. Accordingly, various products by functionalization using surface treatment technology have been proposed so far. Incidentally, the term "touch-proof" or "touch-proof properties" means an effect that a cosmetic film does not easily come off even if a finger, handkerchief, or the like touches the cosmetic film.

As a material for powder formulation having excellent moisture retention, skin adhesion, safety, etc., Lipidure (registered trademark of NOF Corporation), which is a methacryloyloxyethyl phosphorylcholine (hereinafter, abbreviated to "MPC") polymer including MPC as a repeating unit, is known. MPC having a phosphorylcholine group has excellent biocompatibility (bioaffinity) and moisture retention and is used as a biocompatible material, for example, as an artificial organ, a biological membrane, a coating agent for medical equipment, a drug delivery agent, or a cosmetic ingredient. Incidentally, silicone, which has properties that are essential to cosmetic raw materials, i.e., being physiologically inactive, very safe, and stable, has been used for various purposes in the cosmetic field in both solid and liquid (silicone oil) states. For example, a copolymer including, as a repeating unit, silicone methacrylate (SiMA) having a main chain of an acrylic polymer and a modification group of a silicone portion is known to be excellent in water resistance, oil resistance, abrasion resistance, and makeup durability.

As cosmetics containing a polymer having properties of both a polymer including a phosphorylcholine group and silicone, for example, cosmetics containing a polymer including an organopolysiloxane residue and a phosphorylcholine group have been proposed (Patent Documents 1 to 3).

In addition, Patent Document 1 also discloses a polymer further including an unsaturated carboxylic acid and a derivative monomer thereof (for example, cyclohexyl (meth)acrylate or polyoxyalkylene mono(meth)acrylate) as a repeating unit in addition to the organopolysiloxane residue and the phosphorylcholine group. Further, for example, Patent Documents 4 to 6 describe the use of cyclohexyl (meth)acrylate as one component of a copolymer that is used for manufacturing cosmetics. For example, Patent Documents 7 to 10 describe the use of polyoxyalkylene mono(meth)acrylate as one component of a copolymer that is used for manufacturing cosmetics.

### Prior Art Documents

### Patent Documents

[Patent Document 1] International Patent Publication No. WO 2005/094758
[Patent Document 2] International Patent Publication No. WO 96/32436
[Patent Document 3] Japanese Patent No. 2533772
[Patent Document 4] Japanese Unexamined Patent Application Publication (Translation of PCT Application) No. 2004-512292
[Patent Document 5] Japanese Unexamined Patent Application Publication (Translation of PCT Application) No. 2004-531609
[Patent Document 6] Re-publication of PCT International Publication No. WO 2005/032500 (A1)
[Patent Document 7] Japanese Unexamined Patent Application Publication No. 8-291206
[Patent Document 8] Japanese Unexamined Patent Application Publication No. 11-92346
[Patent Document 9] Japanese Unexamined Patent Application Publication (Translation of PCT Application) No. 2008-508384
[Patent Document 10] Japanese Patent No. 4786395
[Patent Document 11] Japanese Unexamined Patent Application Publication No. 2013-139400
[Patent Document 12] Japanese Unexamined Patent Application Publication No. 2004-182729
[Patent Document 13] Japanese Unexamined Patent Application Publication No. 2015-124220
[Patent Document 14] Japanese Unexamined Patent Application Publication No. 2016-210744

### Summary of the Invention

### Object to be Solved by the Invention

In cosmetics, in order to achieve required functions, i.e., durability of makeup and a good touch feeling, it is desired to develop materials for cosmetics having both skin affinity and lubricity. In addition, materials for cosmetics are desired to be excellent in solubility in low polar oils (for example, isododecane, isopropyl alcohol, and a mixture thereof) that are used in cosmetics. From such a viewpoint, the present inventors have aimed to develop a copolymer having all of the following properties:
(1) having both skin affinity and lubricity,
(2) being excellent in water resistance, oil resistance, scratch resistance (abrasion resistance), and makeup durability; and
(3) being excellent in solubility in a low polar oil.

In addition, in cosmetics containing a powder, powder modification technology plays an important role for improving the function and touch feeling. In order to achieve required functions in cosmetics, i.e., durability of makeup and a good touch feeling, it is desired to develop powder treatment agents having both skin affinity and lubricity. In addition, when a powder is treated, a low polar oil (for example, isododecane, isopropyl alcohol, or a mixture thereof) is used as the solvent from the viewpoint of eliminating the powder cohesion during the treatment and improving the drying efficiency in the powder treatment process. It is therefore necessary that the powder treatment agent is excellent in solubility in a low polar oil. From such a viewpoint, the present inventors have aimed to develop a surface-treated powder having all of the following properties:
(1) having both skin affinity and lubricity,
(2) being excellent in water resistance, oil resistance, scratch resistance, and makeup durability,
(3) being excellent in solubility in a low polar oil, and
(4) being excellent in attachment of a surface treatment agent to a powder.

From the above-mentioned viewpoints, the present inventors have diligently examined and studied to develop a copolymer and a powder suitable for being used in cosmetics. As a result, the present inventors found that a copolymer including the four monomers described below as repeating units is significantly excellent in solubility in a low polar oil and that both a good touch feeling and a high function are satisfied by using the copolymer and a powder surface-treated with the copolymer in cosmetics. The present invention has been accomplished based on these findings.

That is, the present invention, defined in the appended claims, includes the following: A copolymer comprising the following (i) to (iv) as repeating units:
(i) methacryloyloxyethyl phosphorylcholine represented by the following formula or a derivative thereof: 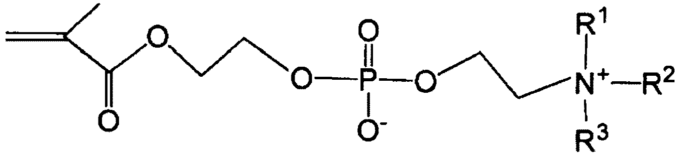 (wherein R¹, R², and R³ are the same as or different from each other and each represent a substituted or unsubstituted linear or branched alkyl group having 1 to 30 carbon atoms);
(ii) a silicone methacrylate represented by the following formula: 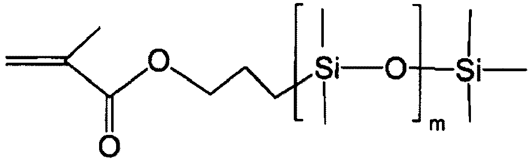 (wherein m represents an integer of 1 to 30);
(iii) a methacrylate represented by the following formula: 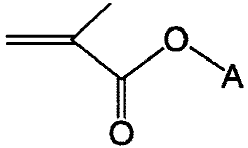 (wherein A is selected from the group consisting of substituted or unsubstituted linear or branched alkyl groups having 1 to 30 carbon atoms, substituted or unsubstituted linear or branched alkenyl groups having 2 to 30 carbon atoms, and substituted or unsubstituted cycloalkyl groups having 3 to 20 carbon atoms); and
(iv) a methoxypolyethylene glycol methacrylate represented by the following formula: 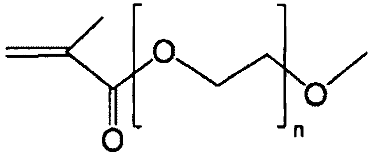 (wherein n represents an integer of 2 to 30).

An oil dispersion comprising the copolymer described above and an oil agent. A cosmetic comprising the copolymer described above.

A surface-treated powder coated with the copolymer described above.

A cosmetic comprising the surface-treated powder described above.

A method for manufacturing the surface-treated powder described above, comprising the following steps (a) to (d):
(a) dissolving the copolymer described above in a solvent with a stirring kneading machine;
(b) adding a powder to the resulting solution and kneading a mixture of the powder and the solution in a form of slurry;
(c) kneading the mixture while removing the solvent in vacuum and taking out the resulting powder; and
(d) drying and then pulverizing the taken-out powder.

### Effect of the Invention

The monomers of (i) to (iv) constituting the copolymer have the following properties. The methacryloyloxyethyl phosphorylcholine and a derivative thereof of (i) and the silicone methacrylate of (ii) are excellent in bioaffinity, moisture retention, water resistance, oil resistance, abrasion resistance, and makeup durability. The methacrylate of (iii) is excellent in solubility in low polar solvents such as isopropyl alcohol, isododecane, and a mixture of isopropyl alcohol and isododecane. The methoxypolyethylene glycol methacrylate of (iv) has high adhesiveness to powder and can uniformly treat powder surfaces. Accordingly, cosmetics including the copolymer of the present invention containing the monomers of (i) to (iv) as repeating units and cosmetics including the surface-treated powder of the present invention prepared by using the copolymer of the present invention as a surface treatment agent have extremely advantageous effects as cosmetics, i.e., being excellent in skin affinity, having a makeup-lasting effect, and also being absence of sense of burden on skin.

### Mode of Carrying Out the Invention

The copolymer in the present invention contains the following (i) to (iv) as repeating units.
(i) Methacryloyloxyethyl phosphorylcholine represented by the following formula or a derivative thereof: 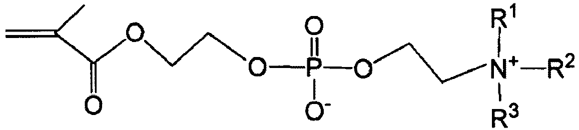 (wherein R¹, R², and R³ are the same as or different from each other and each represent a substituted or unsubstituted linear or branched alkyl group having 1 to 30 carbon atoms).

Although carbon and hydrogen are omitted in the above compound, when carbon and hydrogen are displayed, the compound is as follows, and both mean the same compound. 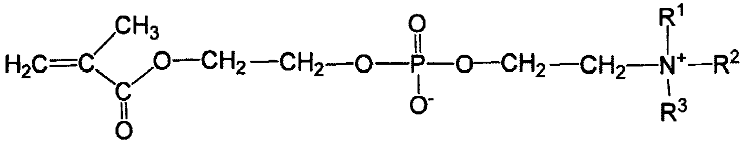 (wherein R¹, R², and R³ are the same as or different from each other and each represent a substituted or unsubstituted linear or branched alkyl group having 1 to 30 carbon atoms).

In addition, the repeating unit of the compound of (i) is represented as follows: 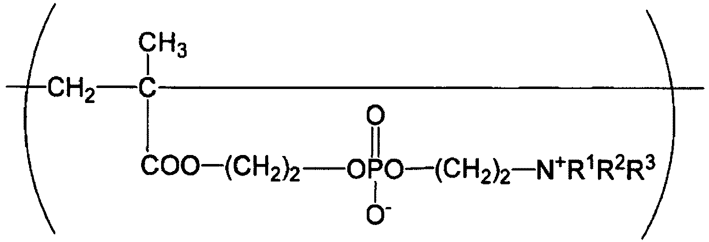 (wherein R¹, R², and R³ are the same as or different from each other and each represent a substituted or unsubstituted linear or branched alkyl group having 1 to 30 carbon atoms).

In each of the above formulae, R¹, R², and R³ are the same as or different from each other and each represent a substituted or unsubstituted linear or branched alkyl group having 1 to 30 carbon atoms. The number of carbon atoms of the substituted or unsubstituted linear or branched alkyl group included in this range is, for example, 1 to 30, 1 to 25, 1 to 20, 1 to 16, 1 to 12, 1 to 10, 1 to 8, 1 to 6, 1 to 4, or 1 to 3. Specifically, examples of the alkyl group include a methyl group, an ethyl group, a propyl group, an isopropyl group, a butyl group, an s-butyl group, an isobutyl group, a t-butyl group, a pentyl group, a hexyl group, a heptyl group, an octyl group, a nonyl group, a decyl group, an undecyl group, and a dodecyl group. In the present specification, in alkyl groups having the same number of carbon atoms, "n-" standing for a linear alkyl group is omitted. For example, "butyl group" means "n-butyl group" .

Examples of the substituent in the substituted linear or branched alkyl group having 1 to 30 carbon atoms include halogen atoms (F, Cl, Br, and I), unsubstituted linear or branched alkyl groups having 1 to 4 carbon atoms, unsubstituted linear or branched alkoxy groups having 1 to 4 carbon atoms, unsubstituted linear or branched alkenyl groups having 2 to 4 carbon atoms, unsubstituted cycloalkyl groups having 3 to 6 carbon atoms, a hydroxyl group, and an amino group.

In one aspect of the combination of R¹, R², and R³, R¹, R², and R³ each represent a methyl group. The compound of (i) in which R¹, R², and R³ each represent a methyl group is methacryloyloxyethyl phosphorylcholine (MPC) represented by the following formula:

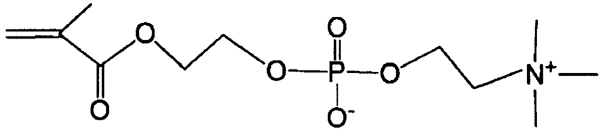

In another aspect of the combination of R¹, R², and R³, R¹, R², and R³ each represent an ethyl group. In another aspect of the combination of R¹, R², and R³, R¹, R², and R³ each represent a propyl group.

(ii) A silicone methacrylate (hereinafter, may be abbreviated to "SiMA") represented by the following formula: 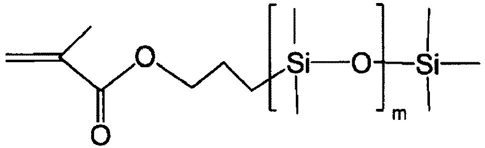 (wherein m represents an integer of 1 to 30).

Although carbon and hydrogen are omitted in the above compound, when carbon and hydrogen are displayed, the compound is as follows, and both mean the same compound. 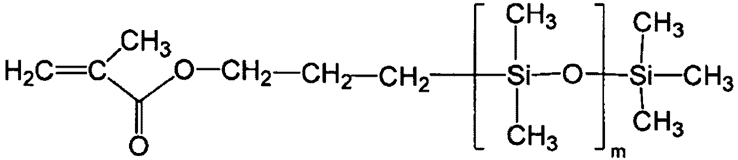 (wherein m represents an integer of 1 to 30).

In addition, the repeating unit of the compound of (ii) is represented as follows: 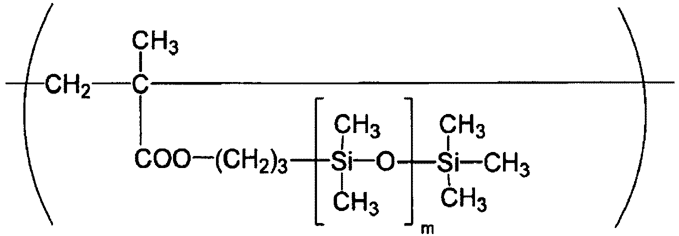 (wherein m represents an integer of 1 to 30).

In each of the above formulae, m represents an integer of 1 to 30. The m included in this range is, for example, an integer of 1 to 30, an integer of 1 to 24, an integer of 1 to 20, an integer of 1 to 18, an integer of 1 to 16, an integer of 1 to 12, an integer of 6 to 24, an integer of 6 to 20, an integer of 6 to 18, an integer of 6 to 16, an integer of 6 to 12, an integer of 6 to 12, an integer of 8 to 24, an integer of 8 to 20, an integer of 8 to 16, an integer of 8 to 12, 8, 9, 10, 11, or 12. The monomer when m is 11 is represented by the following formula:

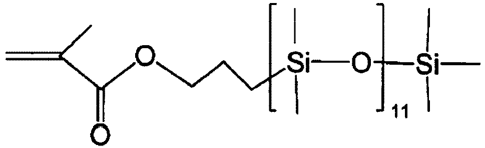

(iii) A methacrylate represented by the following formula: 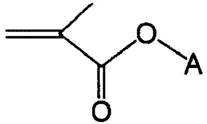 (wherein A is selected from the group consisting of substituted or unsubstituted linear or branched alkyl groups having 1 to 30 carbon atoms, substituted or unsubstituted linear or branched alkenyl groups having 2 to 30 carbon atoms, and substituted or unsubstituted cycloalkyl groups having 3 to 20 carbon atoms and is preferably a substituted or unsubstituted cycloalkyl group having 4 to 10 carbon atoms).

Although carbon and hydrogen are omitted in the above compound, when carbon and hydrogen are displayed, the compound is as follows, and both mean the same compound. 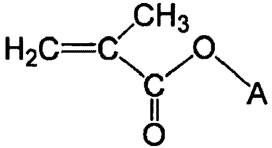 (wherein A is selected from the group consisting of substituted or unsubstituted linear or branched alkyl groups having 1 to 30 carbon atoms, substituted or unsubstituted linear or branched alkenyl groups having 2 to 30 carbon atoms, and substituted or unsubstituted cycloalkyl groups having 3 to 20 carbon atoms and is preferably a substituted or unsubstituted cycloalkyl group having 4 to 10 carbon atoms).

In addition, the repeating unit of the compound of (iii) is represented as follows: 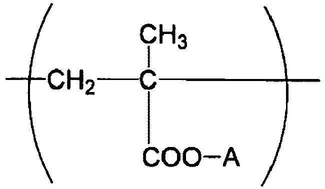 (wherein A is selected from the group consisting of substituted or unsubstituted linear or branched alkyl groups having 1 to 30 carbon atoms, substituted or unsubstituted linear or branched alkenyl groups having 2 to 30 carbon atoms, and substituted or unsubstituted cycloalkyl groups having 3 to 20 carbon atoms and is preferably a substituted or unsubstituted cycloalkyl group having 4 to 10 carbon atoms).

In each of the above formulae, A is selected from the group consisting of substituted or unsubstituted linear or branched alkyl groups having 1 to 30 carbon atoms, substituted or unsubstituted linear or branched alkenyl groups having 2 to 30 carbon atoms, and substituted or unsubstituted cycloalkyl groups having 3 to 20 carbon atoms and is preferably a substituted or unsubstituted cycloalkyl group having 4 to 10 carbon atoms.

The number of carbon atoms of the substituted or unsubstituted linear or branched alkyl group included in the above range is, for example, 1 to 30, 1 to 25, 1 to 20, 1 to 16, 1 to 12, 1 to 10, 1 to 8, 1 to 6, 1 to 4, or 1 to 3. Specifically, examples of the alkyl group include a methyl group, an ethyl group, a propyl group, an isopropyl group, a butyl group, an s-butyl group, an isobutyl group, a t-butyl group, a pentyl group, a hexyl group, a heptyl group, an octyl group, a 2-ethylhexyl group, a nonyl group, a decyl group, an undecyl group, and a dodecyl group (lauryl group).

The number of carbon atoms of the substituted or unsubstituted linear or branched alkenyl group included in the above range is, for example, 2 to 30, 2 to 25, 2 to 20, 2 to 16, 2 to 12, 2 to 10, 2 to 8, 2 to 6, or 2 to 4. Specifically, examples of the alkenyl include an ethenyl group, a propenyl group, a butenyl group, a pentenyl group, a hexenyl group, a heptenyl group, an octenyl group, a nonenyl group, a decenyl group, an undecenyl group, and a dodecenyl group.

The number of carbon atoms of the substituted or unsubstituted cycloalkyl group included in the above range (when a substituent is present, the number of carbon atoms of the substituent is excluded) is, for example, 3 to 20, 3 to 16, 3 to 12, 3 to 8, or 3 to 6. Specifically, examples of the cycloalkyl group include a cyclopropyl group, a cyclobutyl group, a cyclopentyl group, a cyclohexyl group, a cycloheptyl group, and a cyclooctyl group.

Examples of the substituent in the substituted linear or branched alkyl group having 1 to 30 carbon atoms, the substituted linear or branched alkenyl group having 2 to 30 carbon atoms, and the substituted cycloalkyl group having 3 to 20 carbon atoms include halogen atoms (F, Cl, Br, and I), unsubstituted linear or branched alkyl groups having 1 to 4 carbon atoms, unsubstituted linear or branched alkoxy groups having 1 to 4 carbon atoms, unsubstituted linear or branched alkenyl groups having 2 to 4 carbon atoms, unsubstituted cycloalkyl groups having 3 to 6 carbon atoms, a hydroxyl group, and an amino group.

Specifically, examples of A include a 2-ethylhexyl group, a lauryl group, and a cyclohexyl group. The compound when A is a cyclohexyl group is cyclohexyl methacrylate (hereinafter, abbreviated to "CHMA") represented by the following formula:

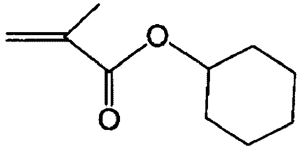

(iv) A methoxypolyethylene glycol methacrylate (hereinafter, may be abbreviated to "PME") represented by the following formula: 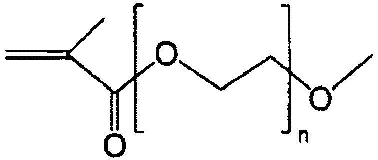 (wherein n represents an integer of 2 to 30).

Although carbon and hydrogen are omitted in the above compound, when carbon and hydrogen are displayed, the compound is as follows, and both mean the same compound. 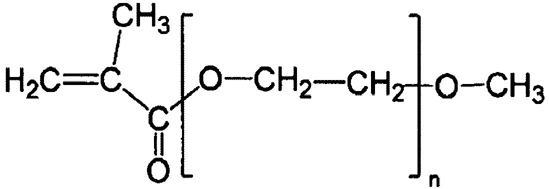 (wherein n represents an integer of 2 to 30).

In addition, the repeating unit of the compound of (iv) is represented as follows: 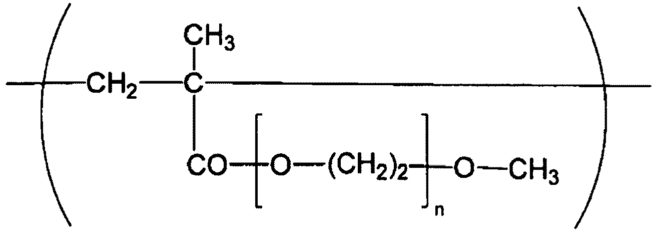 (wherein n represents an integer of 2 to 30).

In each of the above formulae, n represents an integer of 2 to 30. For example, n represents an integer of 2 to 25. For example, n represents an integer of 2 to 20. For example, n represents an integer of 2 to 16. For example, n represents an integer of 2 to 12. For example, n represents an integer of 2 to 9. For example, n represents an integer of 2 to 6. For example, n represents an integer of 2 to 4. For example, n represents 2. For example, n represents 3. For example, n represents 4. For example, n represents 6. For example, n represents 8. For example, n represents 9. For example, n represents 10. For example, n represents 21. For example, n represents 22. For example, n represents 23. For example, n represents 24. For example, n represents 25.

The content rates of (i), (ii), (iii), and (iv) constituting the copolymer of the present invention and the content rates of (i), (ii), (iii), and (iv) constituting the copolymer that is used in surface treatment of the present invention (hereinafter, may be abbreviated as "copolymer") are not particularly limited, but are the following rates.

The copolymer contains (i) in an amount of 0.5 to 10 mass% based on the copolymer mass. Examples of the mass% of (i) within this range include 0.5 to 10 mass%, 2 to 8 mass%, 2.5 to 5.5 mass%, and 5 to 5.5 mass%.

The copolymer contains (ii) in an amount of 0.5 to 10 mass% based on the copolymer mass. Examples of the mass% of (ii) within this range include 0.5 to 10 mass%, 2.5 to 10 mass%, 5 to 10 mass%, 5 to 5.5 mass%, and 2.5 to 5.5 mass%.

The copolymer contains (iii) in an amount of 75 to 90 mass% based on the copolymer mass. Examples of the mass% of (iii) within this range include 75 to 90 mass%, 80 to 90 mass%, 85 to 90 mass%, and 87 to 90 mass%.

The copolymer contains (iv) in an amount of 1 to 5 mass% based on the copolymer mass. Examples of the mass% of (iv) within this range include 1 to 5 mass%, 1 to 3 mass%, and 1 to 2.5 mass%.

The molecular weight (weight-average molecular weight (Mw)) of the copolymer of the present invention and the molecular weight (weight-average molecular weight (Mw)) of the copolymer that is used in surface treatment of the present invention are not particularly limited as long as the purpose of the present invention can be achieved, but are preferably 10,000 to 150,000. Examples of the molecular weight within this range include 10,000 to 120,000, 10,000 to 100,000, 10,000 to 80,000, 10,000 to 70,000, 10,000 to 50,000, 20,000 to 150,000, 20,000 to 120,000, 20,000 to 100,000, 20,000 to 80,000, 20,000 to 70,000, 20,000 to 50,000, 30,000 to 150,000, 30,000 to 120,000, 30,000 to 100,000, 30,000 to 80,000, 30,000 to 70,000, 30,000 to 50,000, 40,000 to 150,000, 40,000 to 120,000, 40,000 to 100,000, 40,000 to 80,000, 40,000 to 70,000, 50,000 to 150,000, 50,000 to 120,000, 50,000 to 100,000, and 50,000 to 80,000. The weight-average molecular weight of the copolymer of the present invention is calculated by a multi-angle light scattering method (MALS method) using a multi-angle light scattering detector. When the weight-average molecular weight of the copolymer of the present invention is less than 10,000, the film-forming properties are slightly inferior, and when the weight-average molecular weight is higher than 150,000, the solubility in a low polar oil is slightly inferior. Thus, both are not preferred.

The copolymer including (i) to (iv) as repeating units of the present invention and the copolymer that is used in surface treatment of the present invention can be prepared by random polymerization through a known polymerization method in the presence of an organic solvent (in the absence of water). Although not particularly limited, the polymerization may be performed in the presence of a radical polymerization initiator, for example, an organic peroxide, such as t-butyl peroxyneodecanoate, benzoyl peroxide, or lauroyl peroxide; an azo compound, such as α,α'-azobisisobutyronitrile, 2,2'-azobis(2,4-dimethylvaleronitrile), or 2,2'-azobis(2-methylbutyronitrile); or a persulfate polymerization initiator, such as potassium persulfate or ammonium persulfate. These polymerization initiators may be used alone or may be used as a mixture of two or more thereof. The amount of the polymerization initiator used is usually 0.01 to 10 parts by mass, preferably 0.1 to 5 parts by mass, based on 100 parts by mass of the monomer composition.

The polymerization may be performed in an atmosphere of an inert gas such as nitrogen. In addition, for example, a solution polymerization method, a suspension polymerization method, a bulk polymerization method, or a precipitation polymerization method can be used. Among these methods, in particular, the solution polymerization method is preferred because of its ease in adjustment of the molecular weight of the resulting acrylic copolymer to an optimum range. In addition, the copolymer of the present invention and the copolymer that is used in surface treatment of the present invention can be purified by a known purification method, such as a reprecipitation method or a dialysis method. The copolymer of the present invention may be any of a random copolymer, a block copolymer, and a graft copolymer.

Examples of the organic solvent used in polymerization of the copolymer of the present invention and the copolymer that is used in surface treatment of the present invention include aromatic hydrocarbons, such as benzene, toluene, and xylene; ketones, such as methyl ethyl ketone and methyl isobutyl ketone; esters, such as ethyl acetate and butyl acetate; and alcohols, such as isopropanol, ethanol, and methanol. These solvents may be used alone or in a combination of two or more thereof. In addition, the polymerization can also be performed in a paraffin solvent, such as light isoparaffin, isododecane, or isohexadecane.

The polymerization reaction temperature of the copolymer of the present invention and the copolymer that is used in surface treatment of the present invention is not particularly limited within a range in which usual radical polymerization initiators can be used, and the polymerization is usually carried out in a range of 40°C to 120°C. The reaction temperature varies depending on the radical polymerization initiator to be used, the type of the monomer, and the reaction temperature, and the polymerization is usually carried out for 2 to 24 hours. If the polymerization time is too short, unpreferably, the amount of residual monomer is large, resulting in a reduction of the yield.

The copolymer of the present invention and the copolymer that is used in surface treatment of the present invention may remain dissolved in the paraffin solvent used in the reaction or may be diluted with another hydrocarbon, ester, or oil agent such as triglyceride as needed or may be subjected to solvent displacement with another oil agent. Such a copolymer composition in an-oil dissolved form is also included in the present invention. In addition, the copolymer can be taken out as a solid by removing the solvent from the solution, and further, the resulting copolymer can also be used as a copolymer solution by being dissolved in light isoparaffin. The above copolymers and the solutions thereof may be used as a mixture of two or more.

One of remarkable characteristics of the copolymer of the present invention and the copolymer that is used in surface treatment of the present invention is significantly high solubility in a low polar solvent. Examples of the low polar solvent include benzene, toluene, n-hexane, cyclohexane, diethyl ether, methylene chloride, chloroform, ethyl acetate, tetrahydrofuran, isopropyl alcohol, and isododecane (2,2,4,6,6-pentamethylheptane). However, the low polar solvent that is used in cosmetics is required to, for example, be very stable, hardly cause a change with time and a chemical change, be low in skin irritation, have low variation in quality, and show uniform drying properties. Examples of the low polar solvent satisfying these requirements include isopropyl alcohol, isododecane, and mixtures thereof (for example, a mixture of isopropyl alcohol and isododecane at a ratio of 1:2).

The copolymer that is used in surface treatment of the present invention itself can be used as a raw material for manufacturing various cosmetics. In manufacturing of a cosmetic using the copolymer of the present invention, other components that are usually used in cosmetics, such as a volatile component, a surfactant, an oil agent, a powder, an aqueous component, a water-soluble polymer, an ultraviolet absorber, a moisturizing agent, a fading inhibitor, an antioxidant, an antifoaming agent, a skin-beautifying component, a preservative, and perfume, can be appropriately used for providing various effects to the copolymer. In addition, the surface-treated powder of the present invention can be used. The surface-treated powder is excellent in skin adhesion and lubricity and is of extremely high quality as a powder formulation and therefore can be applied to manufacturing of various cosmetics.

The oil dispersion prepared by adding the copolymer of the present invention to an oil agent can also be used as a raw material for manufacturing cosmetics. Any oil agent that is usually used in cosmetics can be used without specific limitation. The properties are not particularly limited, but an oil agent being a liquid at ordinary temperature (15°C to 25°C) is preferred.

The oil agent may be of any origin such as animal oil, plant oil, and synthetic oil, and examples thereof include hydrocarbon oils, ester oils (in particular, ester oils having zero or one hydroxyl group), fatty acids, silicone oils, and fluorine-based oils. Among these oil agents, since the strength of a polyurethane gel composition tends to increase with a decrease in polarity, for example, one or more oils selected from hydrocarbon oils, ester oils having zero or one hydroxyl group, and silicone oils containing a phenyl group is used. Specifically, examples of the oil agent include hydrocarbons, such as liquid paraffin, squalane, polybutene, and polyisobutylene; fats and oils, such as olive oil, castor oil, mink oil, and macadamia nut oil; esters, such as jojoba oil, cetyl isooctanoate (cetyl 2-ethylhexanoate), isopropyl myristate, isopropyl palmitate, octyldodecyl myristate, glyceryl trioctanoate, polyglyceryl diisostearate, diglyceryl isostearate, diisostearyl malate, glyceryl tribehenate, pentaerythritol rosinate, and neopentyl glycol dioctanoate; methylphenylpolysiloxane, and diphenylpolysiloxane. One or more of these oil agents can be used.

The content of the oil agent varies depending on the type of the cosmetic, the purpose of use, and so on. The content of the oil agent is, for example, 65 to 99 mass%, 70 to 95 mass%, 75 to 90 mass%, or 80 to 85 mass% based on the oil dispersion mass.

All of the copolymer itself of the present invention, the oil dispersion prepared by adding the copolymer of the present invention to an oil agent, and the surface-treated powder coated with the copolymer of the present invention can be used as a raw material for manufacturing various cosmetics, from skin care formulations to makeup formulations, and also can be applied to any dosage form, such as powder cosmetics, oily cosmetics, aqueous cosmetics, and emulsion cosmetics and can be applied to any shape of the dosage form, such as powders, liquids, and solids. For example, it is possible to apply to manufacturing of a broad range of cosmetics, for example, basic cosmetics, such as lotion, emulsion, cream, oil cleansing, cleansing cream, body milk, and sunscreen; hair cosmetics, such as styling water and hair wax; lip gloss, rouge, eye shadow, foundation, face color, concealer, makeup base, makeup remover, and eyeliner, and powder cosmetics such as loose powder; and makeup cosmetics, such as foundations such as powder foundation and liquid foundation, concealer, eye shadow, face color, mascara, and eyeliner. Incidentally, in manufacturing of a cosmetic using the copolymer itself of the present invention, the oil dispersion prepared by adding the copolymer of the present invention to an oil agent, or the surface-treated powder coated with the copolymer of the present invention, in addition to each of them, other components that are usually used in cosmetics for providing various effects, such as a volatile component, a surfactant, an oil agent, a powder, an aqueous component, alcohols, a water-soluble polymer, an ultraviolet absorber, a moisturizing agent, a gelling agent and thickener, a fading inhibitor, an antioxidant, an antifoaming agent, a skin-beautifying component (whitening agent, cell activating agent, anti-inflammatory agent, blood circulation promoter, skin astringent, antiseborrheic agent, etc.), a preservative, an antibacterial agent, perfume, vitamins, amino acids, a nucleic acid, and a hormone, can be appropriately used.

As obvious from the following Examples, it was proved that the cosmetics, such as lotion, emulsion, cream, oil cleansing, cleansing cream, styling water, foundation, face color, face powder, concealer, sunscreen, makeup base, body milk, makeup remover, and eyeliner, manufactured using the copolymer of the present invention, the oil dispersion prepared by adding the copolymer of the present invention to an oil agent, or the surface-treated powder coated with the copolymer of the present invention are excellent in "affinity to skin", "makeup-lasting effect", and "absence of sense of burden on skin". It was proved that the eye shadow manufactured using the copolymer of the present invention is excellent in "affinity to eyelids", "makeup-lasting effect", and "absence of sense of burden on eyelids". It was proved that the mascara manufactured using the copolymer of the present invention is excellent in "affinity to eyelashes", "makeup-lasting effect", and "absence of sense of burden on eyelashes". It was proved that the hair wax manufactured using the copolymer of the present invention is excellent in "affinity to hair", "makeup-lasting effect", and "absence of sense of burden on hair". It was proved that the lip gloss and rouge manufactured using the copolymer of the present invention are excellent in "affinity to lips", "makeup-lasting effect", and "absence of sense of burden on lips".

Examples of other components include an aqueous component, surfactants (anionic surfactant, cationic surfactant, nonionic surfactant, amphoteric surfactant, etc.), a moisturizing agent, a thickener, a gelling agent, a film-forming agent, a coloring agent, a powder other than the coloring agent, oil agents (solid oil, semi-solid oil, liquid oil, hydrocarbon oil, ester oil, glyceride oil, silicone oil, etc.), alcohols including higher alcohol, higher fatty acid, an organic solvent, a skin-beautifying component, a preservative, an antibacterial agent, perfume, an antioxidant, vitamins, amino acids, and an ultraviolet absorber. These components will be each described below.

The aqueous component is not particularly limited and may be any aqueous component that is a liquid at ordinary temperature and is usually used in cosmetics, and examples thereof include water, ethanol, polyhydric alcohols, such as propylene glycol, 1,3-butylene glycol, dipropylene glycol, and tripropylene glycol; glycerins, such as glycerin, diglycerin, and polyglycerin; and extracts of plants, such as aloe vera, hamamelis, cucumber, lemon, lavender, and rose. One or more of these aqueous components can be used as needed. The content of the aqueous component used in the present invention varies depending on the usability, the sense of use, and the dosage form and is not particularly limited, but is preferably 10 to 90 mass%, in particular, 20 to 75 mass%, in all components from the viewpoint of the makeup effect, usability, and sense of use.

The surfactant may be any surfactant and is not particularly limited as long as it is usually used in cosmetics. Examples of the surfactant include anionic surfactants, cationic surfactants, nonionic surfactants, and amphoteric surfactants, and these surfactants can be used alone or in a combination of two or more thereof as needed.

Specifically, examples of the anionic surfactant include fatty acid soap, such as sodium stearate and triethanolamine palmitate, alkyl ether carboxylic acids and salts thereof, carboxylates by, for example, condensation of amino acids and fatty acids, alkyl sulfonic acids, alkene sulfonates, sulfonates of fatty acid esters, sulfonates of fatty acid amides, sulfonates of alkyl sulfonates and formalin condensates thereof, alkyl sulfate ester salts, secondary higher alcohol sulfate ester salts, alkyl and ally ether sulfate ester salts, fatty acid ester sulfate ester salts, sulfate ester salts of fatty acid alkylolamide, polyoxyethylene alkyl sulfate ester salts, sulfate ester salts, such as sulfonated oil, alkyl phosphates, ether phosphates, alkyl allyl ether phosphates, amidophosphates, and N-acylamino acid-based surfactants.

Examples of the cationic surfactant include alkyl quaternary ammonium salts and aromatic quaternary ammonium salts, such as a long-chain alkyltrimethylammonium salt, a di-long-chain alkyldimethylammonium salt, a long-chain alkyldimethylbenzylammonium salt, a dipolyoxyethylene alkylmethylammonium salt, a dipolyoxyethylene alkyl ether dimethylammonium salt, and a polyoxypropylene methyldiethylammonium salt and also include pyridinium salts, such as an alkylpyridinium salt, imidazoline salts, such as an alkyl dihydroxyethyl imidazoline salt, N-acyl basic amino acid lower alkyl ester salts, and amine salts, such as an alkylamine salt, a polyamine, and an amino alcohol fatty acid derivative.

Examples of the nonionic surfactant include sorbitan fatty acid ester, glycerin fatty acid ester, polyglycerin fatty acid ester, propylene glycol fatty acid ester, polyethylene glycol fatty acid ester, sucrose fatty acid ester, polyoxyethylene alkyl ether, polyoxypropylene alkyl ether, polyoxyethylene alkyl phenyl ether, polyoxyethylene fatty acid ester, polyoxyethylene sorbitan fatty acid ester, polyoxyethylene sorbitol fatty acid ester, polyoxyethylene glycerin fatty acid ester, polyoxyethylene propylene glycol fatty acid ester, polyoxyethylene castor oil, polyoxyethylene hydrogenated castor oil, polyoxyethylene hydrogenated castor oil fatty acid ester, polyoxyethylene phytostanol ether, polyoxyethylene phytosterol ether, polyoxyethylene cholestanol ether, polyoxyethylene cholesteryl ether, polyoxyalkylene-modified organopolysiloxane, polyoxyalkylene-alkyl co-modified organopolysiloxane, alkanolamide, sugar ether, and sugar amide.

Examples of the amphoteric surfactant include carbobetaine-type amphoteric surfactants, such as alkyldimethylaminoacetic acid betaine, fatty acid amide propyldimethylaminoacetic acid betaine, and alkyldihydroxyethylaminoacetic acid betaine; sulfobetiane-type amphoteric surfactants, such as alkyl sulfobetaine; amidoamine-type (imidazoline-type) amphoteric surfactants, such as an N-fatty acid acyl-N-carboxymethyl-N-hydroxyethylethylenediamine salt and an N-fatty acid acyl-N-carboxymethoxyethyl-N-carboxymethylethylenediamine disalt; amino acid-type amphoteric surfactants, such as an N-[3-alkyloxy-2-hydroxypropyl]arginine salt; and alkyliminodicarboxylate-type amphoteric surfactants.

Examples of the moisturizing agent include urea, hyaluronic acid, chondroitin sulfate, and pyrrolidone carboxylate.

Examples of aqueous thickener and gelling agent include plant-based polymers, such as gum arabic, tragacanth gum, galactan, carob gum, guar gum, karaya gum, carrageenan gum, pectin, agar, quince seed (quince), starch (rice, corn, potato, and wheat), algae colloid, tranto gum, and locust bean gum; microbial polymers, such as xanthan gum, dextran, succinoglucan, and pullulan; animal polymers, such as collagen, casein, albumin and gelatin; starch polymers, such as carboxymethyl starch and methylhydroxypropyl starch; cellulose polymers, such as methyl cellulose, hydroxypropylmethyl cellulose, carboxymethyl cellulose, hydroxymethyl cellulose, hydroxypropyl cellulose, sodium cellulose sulfate, and sodium carboxymethylcellulose; alginic acid polymers, such as sodium alginate and propylene glycol alginate; vinyl polymers, such as sodium polyacrylate, a carboxyvinyl polymer, an alkyl-modified carboxyvinyl polymer, polyacrylamide, polyvinyl alcohol, and polyvinylpyrrolidone; polyethylene glycols; ethylene oxide-propylene oxide copolymers; acrylic polymers, such as an acrylic acid-acryloyl dimethyl taurine sodium copolymer, sodium polyacrylate, polyethyl acrylate, and polyacrylamide; inorganic gelling agents and thickeners, such as bentonite, aluminum magnesium silicate, laponite, hectorite, and silicic anhydride; polyethyleneimines; and cationic polymers.

As the film-forming agent, for example, natural rubber, natural cellulose, cationized cellulose, various acrylic resins (including copolymers), various alkyd resins, polyvinyl alcohol, polyvinylpyrrolidone, nitrocellulose, various silicone resins (including copolymers), urea resins, and modified corn starch are used. Specifically, examples of the film-forming agent include an acrylic amide-alkyl acrylate-methacrylic acid methoxypolyethylene glycol copolymer, methacrylic acid methoxypolyethylene glycol, an eicosane-vinylpyrrolidone polymer, a 1,1'-methylenebis(4-isocyanatocyclohexane)polypropylene glycol copolymer, perfluoropolyether, an acrylate-polytrimethylsiloxy methacrylate copolymer, a polypropylsilsesquioxane, eicosane-vinylpyrrolidone copolymer, a vinylpyrrolidone-hexadecane copolymer, hydroxyethyl cellulose, and silicone-modified norbornene.

The coloring agent may be any coloring agent that is usually used in cosmetics and is not particularly limited in shape, such as spherical, tabular, spindle-like, and needle-like shapes, in particle diameter, such as aerosol, microparticle, and pigment class particle diameters, and in particle structure, such as porous and nonporous structures, and, for example, inorganic pigments, organic pigments, bright pigment, and metals can be used. Specifically, examples of the powder include white inorganic pigments, such as titanium oxide, zinc oxide, cerium oxide, and barium sulfate; colored inorganic pigments, such as iron oxide, carbon black, chromium oxide, chromium hydroxide, iron blue, ultramarine blue, and red iron oxide; bright pigments, such as titanated mica, bismuth oxychloride, organic pigment-treated titanated mica, titanium dioxide-coated mica, titanium dioxide-coated synthetic phlogopite, titanium dioxide-coated bismuth oxychloride, iron oxide titanated mica, iron blue-treated titanated mica, carmine-treated titanated mica, argentine, a titanium dioxide-coated glass powder, and resin laminated powders, such as a polyethylene terephthalate-aluminum-epoxy laminated powder, a polyethylene terephthalate-polyolefin laminated film powder, and a polyethylene terephthalate-polymethyl methacrylate laminated film powder; organic pigment powders, such as Red No. 201, Red No. 202, Red No. 205, Red No. 226, Red No. 228, Orange No. 203, Orange No. 204, Blue No. 404, and Yellow No. 401; organic pigment powders of, for example, zirconium, barium, and aluminum lake, such as Red No. 3, Red No. 104, Red No. 106, Orange No. 205, Yellow No. 4, Yellow No. 5, Green No. 3, and Blue No. 1; metal powders, such as an aluminum powder, a gold powder, and a silver powder; composite powders, such as fine titanium oxide particle-coated titanated mica, fine zinc oxide particle-coated titanated mica, barium sulfate-coated titanated mica, titanium oxide-containing silicon dioxide, and zinc oxide-containing silicon dioxide; and natural dyes, such as carminic acid, laccaic acid, calsamine, brazirine, and crocin. One or more of these coloring agents can be used as needed. These coloring agents may be surface-treated with one or more of, for example, a fluorine compound, a silicone compound, metal soap, lecithin, hydrogenated lecithin, collagen, hydrocarbon, higher fatty acid, higher alcohol, ester, wax, and a surfactant.

Powders other than the coloring agent may be any powder that is usually used as a cosmetic raw material and is not particularly limited in shape, such as tabular, spindle-like, and needle-like shapes, in particle diameter, such as aerosol and microparticle class particle diameters, and examples thereof include inorganic powders, such as aluminum oxide, cerium oxide, magnesium oxide, zirconium oxide, magnesium carbonate, potassium carbonate, aluminum silicate, magnesium silicate, aluminum magnesium silicate, mica, synthetic mica, synthetic sericite, sericite, talc, kaolin, silica, silicon carbide, and boron nitride; and organic powders, such as magnesium stearate, zinc stearate, N-acyl lysine, nylon, polymethyl methacrylate, an organopolysiloxane powder, and a cellulose powder. One or more of these powders can be used. In addition, these powders may be used as a composite of one or more thereof or may be subjected to surface treatment such as oil agent treatment, silicone compound treatment, or water-soluble polymer treatment.

Examples of the oil agent include solid oils, semi-solid oils, and liquid oils, such as natural animal and plant oils and semi-synthetic oils, hydrocarbon oils, ester oils, glyceride oils, silicone oils, higher alcohols, higher fatty acid, and organic solvent media.

Examples of the solid oil include natural waxes, such as carnauba wax, candelilla wax, cotton wax, shellac wax, and hydrogenated oil; mineral waxes, such as ozokerite, ceresin, paraffin wax, and microcrystalline wax; synthetic waxes, such as polyethylene wax, Fischer-Tropsch wax, and an ethylene-propylene copolymer; higher alcohols, such as behenyl alcohol, cetyl alcohol, stearyl alcohol, cholesterol, and phytosterol; and higher fatty acids, such as stearic acid and behenic acid.

Examples of the natural animal and plant oil and semi-synthetic oil as the liquid oil include avocado oil, linseed oil, almond oil, insect wax, perilla oil, olive oil, kaya oil, liver oil, apricot kernel oil, wheat germ oil, sesame oil, rice germ oil, rice bran oil, sasanqua oil, safflower oil, China tung oil, cinnamon oil, turtle oil, soybean oil, tea seed oil, camellia oil, primrose oil, corn oil, rapeseed oil, Japan tung oil, bran wax, germ oil, persic oil, palm oil, palm kernel oil, castor oil, sunflower oil, grape oil, jojoba oil, macadamia nut oil, cottonseed oil, coconut oil, tri-coconut oil fatty acid glyceride, peanut oil, lanolin, liquid lanolin, reduced lanolin, lanolin alcohol, lanolin acetate, lanolin fatty acid isopropyl ester, POE lanolin alcohol ether, POE lanolin alcohol acetate, lanolin fatty acid polyethylene glycol, POE hydrogenated lanolin alcohol ether, and egg yolk oil.

Examples of the hydrocarbon oil include squalane, squalene, liquid paraffin, pristane, and polyisobutylene.

Examples of the ester oil include diisobutyl adipate, 2-hexyldecyl adipate, di-2-heptylundecyl adipate, N-alkyl glycol monoisostearate, isocetyl isostearate, trimethylolpropane triisostearate, cetyl 2-ethylhexanoate, ethylene glycol di-2-ethylhexanoate, neopentyl glycol di-2-ethylhexanoate, trimethylolpropane tri-2-ethylhexanoate, pentaerythritol tetra-2-ethylhexanoate, cetyl octanoate, octyldodecyl gum ester, oleyl oleate, octyldodecyl oleate, decyl oleate, neopentyl glycol dicaprate, triethyl citrate, 2-ethylhexyl succinate, amyl acetate, ethyl acetate, butyl acetate, isocetyl stearate, butyl stearate, diisopropyl sebacate, di-2-ethylhexyl sebacate, cetyl lactate, myristyl lactate, isopropyl palmitate, 2-ethylhexyl palmitate, 2-hexyldecyl palmitate, 2-heptylundecyl palmitate, cholesteryl 12-hydroxystearate, dipentaerythritol fatty acid ester, isopropyl myristate, 2-octyldodecyl myristate, 2-hexyldecyl myristate, myristyl myristate, hexyldecyl dimethyloctanoate, ethyl laurate, hexyl laurate, N-lauroyl-L-glutamic acid-2-octyldodecyl ester, and diisostearyl malate.

Examples of the glyceride oil include acetoglyceride, glyceride triisooctanoate, glyceride triisostearate, glyceride triisopalmitate, glyceride tri-2-ethylhexanoate, glyceride monostearate, glyceride di-2-heptylundecanoate, and glyceride trimyristate.

Examples of the silicone oil include dimethylpolysiloxane, methylphenylpolysiloxane, methyl hydrogen polysiloxane, octamethylcyclotetrasiloxane, decamethylcyclopentasiloxane, dodecamethylcyclohexasiloxane, tetramethyl tetrahydrogen cyclotetrasiloxane, and alkyl-modified silicone.

Examples of the higher alcohol include oleyl alcohol, lauryl alcohol, stearyl alcohol, isostearyl alcohol, 2-octyldodecanol, and decyltetradecanol.

Examples of the higher fatty acid include oleic acid, palmitic acid, myristic acid, stearic acid, and isostearic acid.

Examples of the organic solvent include hydrocarbons, such as n-hexane and cyclohexane; aromatic compounds, such as benzene, toluene, and xylene; nonaromatic compounds, such as ethyl acetate and butyl acetate; chlorine compounds, such as chloroform, dichloromethane, and dichloroethane; ether compounds, such as dioxane and tetrahydrofuran; and 2-propanol, benzyl alcohol, phenoxyethanol, carbitols, cellosolves, and spindle oil.

Specifically, the alcohols are, for example, ethanol, isopropanol; polyhydric alcohols, such as erythritol; sugar alcohols, such as sorbitol, maltose, xylitol, and maltitol; and benzyl alcohol.

Examples of the skin-beautifying component include whitening agents, such as arbutin, glutathione, and a saxifrage extract; cell activating agents, such as royal jelly, a photosensitizer, a cholesterol derivative, and a calf blood extract; rough skin-improving agents; blood circulation promoters, such as nonylic acid vanillylamide, nicotinic acid benzyl ester, nicotinic acid β-butoxyethyl ester, capsaicin, zingerone, cantharis tincture, ichthammol, caffeine, tannic acid, α-borneol, tocopherol nicotinate, inositol hexanicotinate, cyclandelate, cinnarizine, tolazoline, acetylcholine, verapamil, cepharanthine, and γ-oryzanol; skin astringents, such as zinc oxide and tannic acid; and antiseborrheic agents, such as sulfur and thianthol.

Examples of the preservative and antibacterial agent include paraoxybenzoic acid ester, benzoic acid, sodium benzoate, sorbic acid, potassium sorbate, phenoxyethanol, salicylic acid, carbolic acid, sorbic acid, parachloromethacresol, hexachlorophene, benzalkonium chloride, chlorhexidine chloride, chlorphenesin, trichlorocarbanilide, a photosensitizer, and isopropylmethylphenol.

In the perfume, natural perfume (animal perfume and plant perfume), synthetic perfume, and compound perfume (a blend of natural perfume and synthetic perfume) are included, and compound perfume prepared by blending natural perfume and synthetic perfume is the most commonly used in cosmetics today. Examples of the perfume include jasmine, tagetes, rosemary, vanilla, ginger oil, rose oil, jasmine oil, lavender oil, ylang ylang oil, peppermint oil, geranium oil, lemon oil, orange oil, star anise oil, grapefruit oil, eucalyptus oil, sandalwood oil, black pepper oil, basil oil, ylang ylang oil, patchouli oil, coumarin, musk ketone, heliotropin, 1-octen-3-ol, and blends thereof.

Examples of the antioxidant include tocopherol, butylhydroxyanisole, and dibutylhydroxytoluene. Examples of pH adjusters include lactic acid, lactate, citric acid, citrate, glycolic acid, succinic acid, tartaric acid, malic acid, potassium carbonate, sodium bicarbonate, and ammonium bicarbonate; examples of chelating agents include alanine, sodium edetate, sodium polyphosphate, sodium metaphosphate, phosphate, and hydroxyethane diphosphone; examples of the refrigerants include L-menthol, camphor, mint oil, peppermint oil, and eucalyptus oil; and examples of the anti-inflammatory agent include allantoin, a glycyrrhetinate, a glycyrrhizin derivative, tranexamic acid, and azulene.

Examples of the vitamins include vitamins A, such as vitamin A oil, retinol, retinol acetate, and retinol palmitate; vitamins B2, such as riboflavin, riboflavin butyrate, and flavin adenine nucleotide; vitamins B6, such as pyridoxine hydrochloride and pyridoxine dioctanoate; vitamins C, such as L-ascorbic acid, L-ascorbic acid dipalmitic acid ester, sodium L-ascorbic acid-2-sulfate, and dipotassium dl-α-tocopherol-L-ascorbic acid phosphoric acid diester; pantothenic acids, such as calcium pantothenate, D-pantothenyl alcohol, pantothenyl ethyl ether, and acetyl pantothenyl ethyl ether; vitamins D, such as ergocalciferol and cholecalciferol; nicotinic acids, such as nicotinic acid, benzyl nicotinate, and nicotinic acid amide; vitamins E, such as dl-α-tocopherol, dl-α-tocopherol acetate, dl-α-tocopherol nicotinate, and dl-α-tocopherol succinate; vitamin P; and biotin.

Examples of the amino acid include arginine, aspartic acid, cystine, cysteine, methionine, serine, leucine, isoleucine, tryptophan, alanine, glycine, and proline; examples of the nucleic acid include deoxyribonucleic acids; and examples of the hormone include estradiol and ethinylestradiol.

Examples of the ultraviolet absorber include benzoic acid ultraviolet absorbers, such as paraaminobenzoic acid; anthranilic acid ultraviolet absorbers, such as methyl anthranilate; salicylic acid ultraviolet absorbers, such as methyl salicylate; cinnamic acid ultraviolet absorbers, such as octyl paramethoxycinnamate; benzophenone ultraviolet absorbers, such as 2,4-dihydroxybenzophenone; urocanic acid ultraviolet absorbers, such as ethyl urocanate; and dibenzoylmethane ultraviolet absorbers, such as 4-t-butyl-4'-methoxy-dibenzoylmethane.

The method for manufacturing the surface-treated powder of the present invention is not particularly limited, and the surface-treated powder can be preferably manufactured by a method including the following steps:
(a) a step of dissolving the copolymer according to the above [1] in a solvent with a stirring kneading machine;
(b) a step of adding a powder to the resulting solution and kneading a mixture of the powder and the solution in a form of slurry;
(c) a step of kneading the mixture while removing the solvent in vacuum and taking out the resulting powder; and
(d) a step of drying and then pulverizing the taken-out powder.

In the step (a), a copolymer including (i) to (iv) described in the above [1] as repeating units is dissolved in a solvent using a stirring kneading machine such as a planetary mixer. The planetary mixer is an all-purpose mixer showing excellent performance in stirring and kneading a solid (powder and granular material) and a liquid and is composed of two frame-like blades, and a shearing force by planetary motion strongly acts due to the precise distances between the blades and between each blade and the tank inner surface to provide the high kneading effect (stirring, kneading, and dispersion). In order to eliminate the powder cohesion during the treatment and improve the drying efficiency, the solvent to be used is desirably a low polar solvent. Examples of such a solvent include isopropyl alcohol (IPA), isododecane, and a mixture of IPA and isododecane. When a mixture of IPA and isododecane is used, the mixing ratio is not particularly limited and is, for example, IPA : isododecane is 1:3 to 3:1. In particular, a mixture of IPA and isododecane in a ratio of 1:2 is more preferably used.

In the step (b), a powder is added to the solution containing the polymer dissolved therein in the step (a), and the resulting mixture is kneaded in a form of slurry. The powder to be used is not particularly limited, and one or more powders selected from minerals and metal oxides are preferred. For example, the mineral and the metal oxide to be used are, for example, sericite, mica, talc, titanium oxide, zinc oxide, and iron oxides (FeO, Fe₂O₃, Fe₃O₄, and a mixture thereof). One or more of these powders are used as needed. Incidentally, the mass ratio of the solvent and the methacryloyloxyethyl phosphorylcholine-silicone methacrylate copolymer and the powder is not particularly limited and is, for example, (solvent) : (methacryloyloxyethyl phosphorylcholine-silicone methacrylate copolymer and powder) is 1:3 to 1:1. As one embodiment, (solvent) : (methacryloyloxyethyl phosphorylcholine-silicone methacrylate copolymer and powder) is 1:2.

In the step (c), the mixture kneaded in the step (b) is kneaded while removing the solvent in vacuum, and the resulting powder is taken out. The vacuum is, for example, 700 to 760 mmHG.

In the step (d), the powder surface-coated with the methacryloyloxyethyl phosphorylcholine-silicone methacrylate copolymer is further dried, and the resulting solid is pulverized by a pulverizer (grinder). Thus, the methacryloyloxyethyl phosphorylcholine-silicone methacrylate copolymer-treated powder of the present invention can be obtained.

The present invention will now be described in further detail with reference to Examples. Incidentally, these examples do not limit the present invention in any way.

### Examples

### [Synthesis Example 1]

5.0 g of 2-Methacryloyloxyethyl phosphorylcholine (MPC, manufactured by NOF Corporation), 5.0 g of silicone methacrylate (SiMA, manufactured by Shin-Etsu Chemical Co., Ltd.), 85.0 g of cyclohexyl methacrylate (Blemmer CHMA, manufactured by NOF Corporation), and 5.0 g of methoxypolyethylene glycol methacrylate (Blemmer PME-1000, manufactured by NOF Corporation) were put in a four-neck flask and were dissolved in 233.3 g of 2-propanol, followed by blowing of nitrogen gas for 30 minutes. Subsequently, 2.0 g of t-butyl peroxyneodecanoate (Perbutyl (Japanese registered trademark) ND100 (PB-ND100), manufactured by NOF Corporation) was added as a polymerization initiator to the flask, and a polymerization reaction was performed at 70°C for 6 hours. After the polymerization reaction, the polymerization solution was dropwise added to an excess amount of ethanol while stirring, and the deposited precipitate was filtered, followed by vacuum drying at room temperature for 48 hours to obtain a powder. The yield amount was 70.0 g. The molecular weight of the polymer was analyzed by an MALS method. The apparatus used was a multi-angle light scattering detector (DAWN HELEOS II 8+), and the medium used was isopropanol. The weight-average molecular weight was 60,000.

### [Synthesis Examples 2 to 11]

Polymers were manufactured according to the same procedure as Synthesis Example 1 except that the components of the types and the amounts shown in Table 1 below were used.

### [Synthesis Comparative Examples 1 to 4]

Polymers were manufactured according to the same procedure as Synthesis Example 1 except that the components of the types and the amounts shown in Table 1 below were used.

**[Table 1]**

| | MPC | SiMA | CHMA | 2-EHMA | RMA | PME1 | PME2 | PME3 | PME4 |
|---|---|---|---|---|---|---|---|---|---|
| Synthesis Example 1 | 5 | 5 | 85 | - | - | 5 | - | - | - |
| Synthesis Example 2 | 2 | 5 | 88 | - | - | 5 | - | - | - |
| Synthesis Example 3 | 8 | 5 | 82 | - | - | 5 | - | - | - |
| Synthesis Example 4 | 5 | 10 | 80 | - | - | 5 | - | - | - |
| Synthesis Example 5 | 2.5 | 10 | 85 | - | - | 2.5 | - | - | - |
| Synthesis Example 6 | 5.1 | 5.1 | 87.4 | - | - | - | 2.4 | - | - |
| Synthesis Example 7 | 5.3 | 5.3 | 88.2 | - | - | - | 1.2 | - | - |
| Synthesis Example 8 | 5.2 | 5.2 | 88.3 | - | - | - | - | 1.3 | - |
| Synthesis Example 9 | 5.2 | 5.2 | 88.7 | - | - | - | - | - | 0.9 |
| Synthesis Example 10 | 5 | 5 | - | 85 | - | - | - | - | 5 |
| Synthesis Example 11 | 5 | 5 | - | - | 85 | - | - | - | 5 |
| Synthesis Comparative Example 1 | 5 | 5 | 90 | - | - | - | - | - | - |
| Synthesis Comparative Example 2 | 5 | - | 90 | - | - | 5 | - | - | - |
| Synthesis Comparative Example 3 | - | 5 | 90 | - | - | 5 | - | - | - |
| Synthesis Comparative Example 4 | 47.5 | 47.5 | - | - | - | 5 | - | - | - |

| | | | | | | | | | |
|---|---|---|---|---|---|---|---|---|---|
| (Note) Abbreviations in Table 1 have the following meanings: MPC: 2-methacryloyloxyethyl phosphorylcholine (manufactured by NOF Corporation), SiMA: silicone methacrylate (manufactured by Shin-Etsu Chemical Co., Ltd.), CHMA: cyclohexyl methacrylate (Blemmer CHMA, manufactured by NOF Corporation), 2-EHMA: 2-ethylhexyl methacrylate (manufactured by Tokyo Chemical Industry Co., Ltd.), RMA: lauryl methacrylate (manufactured by Tokyo Chemical Industry Co., Ltd.), PME 1: methoxypolyethylene glycol methacrylate (number of moles added: 23) (Blemmer PME-1000, manufactured by NOF Corporation), PME 2: methoxypolyethylene glycol methacrylate (number of moles added: 9) (Blemmer PME-400, manufactured by NOF Corporation), PME 3: methoxytetraethylene glycol methacrylate (Blemmer PME-200, manufactured by NOF Corporation), and PME 4: methoxydiethylene glycol methacrylate (Blemmer PME-100, manufactured by NOF Corporation) | | | | | | | | | |

### [Test Example]

### Evaluation of methacryloyloxyethyl phosphorylcholine-silicone methacrylate copolymer

The solubility of methacryloyloxyethyl phosphorylcholine-silicone methacrylate copolymers described in Synthesis Examples 1, 10, and 11 and Synthesis Comparative Example 4 was evaluated.

### (Evaluation method)

Each copolymer and isopropyl alcohol and isododecane as solvents were heated at 80°C, and the transparent dissolution was visually verified. The results are shown in Table 2.

**[Table 2]**

| Component | Test Example | | | | Comparative Test Example | |
|---|---|---|---|---|---|---|
| | 1 | 2 | 3 | 4 | 1 | 2 |
| Copolymer of Synthesis Example 1 | 1 | 1 | - | - | - | - |
| Copolymer of Synthesis Example 10 | - | - | 1 | - | - | - |
| Copolymer of Synthesis Example 11 | - | - | - | 1 | - | - |
| Copolymer of Synthesis Comparative Example 4 | - | - | - | - | 1 | 1 |
| Isopropyl alcohol | 99 | 33 | 33 | 33 | 99 | 33 |
| Isododecane | - | 66 | 66 | 66 | - | 66 |
| (Evaluation item) | | | | | | |
| Solubility | Excellent | Excellent | Excellent | Excellent | Poor | Poor |

| | | | | | | |
|---|---|---|---|---|---|---|
| (Note) In the above table, Excellent and Poor have the following meanings: Excellent: complete dissolution, and Poor: clouded or precipitation of resin. | | | | | | |

The copolymers of Synthesis Examples 2 to 9 were also subjected to the same test as above, and the solubility was visually verified based on the same evaluation method as above. As a result, it was confirmed that the copolymers of Synthesis Examples 2 to 9 were also all Excellent (completely dissolved).

### [Copolymer example]

### W/O type makeup base

A makeup base including the methacryloyloxyethyl phosphorylcholine-silicone methacrylate copolymer described in Synthesis Example 1 and makeup bases including the polymers of Synthesis Comparative Examples 1 to 4 were evaluated for affinity to skin, makeup-lasting effect, and absence of sense of burden on skin. The evaluation method and the evaluation results are shown in Table 3.

**[Table 3]**

| Methacryloyloxyethyl phosphorylcholine-silicone methacrylate copolymer blend basic patent | | | | | | | | |
|---|---|---|---|---|---|---|---|---|
| W/O type base | | | | | | | | |
| Table 3: Makeup base (W/O type) | | | | | | | | (mass%) |
| No. | Component | Copolymer Example | | | Copolymer Comparative Example | | | |
| | | 1 | 2 | 3 | 1 | 2 | 3 | 4 |
| 1 | Triethoxycaprylylsilane (2.0%) treated titanium oxide (Note 1) | 10 | 10 | 10 | 10 | 10 | 10 | 10 |
| 2 | Triethoxycaprylylsilane (2.0%) treated talc | 5 | 5 | 5 | 5 | 5 | 5 | 5 |
| 3 | Triethoxycaprylylsilane (2.0%) treated red iron oxide | 0.25 | 0.25 | 0.25 | 0.25 | 0.25 | 0.25 | 0.25 |
| 4 | Triethoxycaprylylsilane (2.0%) treated yellow iron oxide | 1.5 | 1.5 | 1.5 | 1.5 | 1.5 | 1.5 | 1.5 |
| 5 | Triethoxycaprylylsilane (2.0%) treated black iron oxide | 0.1 | 0.1 | 0.1 | 0.1 | 0.1 | 0.1 | 0.1 |
| 6 | PEG-9 polydimethylsiloxyethyl dimethicone (Note 2) | 1 | 1 | 1 | 1 | 1 | 1 | 1 |
| 7 | Lauryl PEG-9 polydimethylsiloxyethyl dimethicone (Note 3) | 2 | 2 | 2 | 2 | 2 | 2 | 2 |
| 8 | Cyclomethicone | 4 | 4 | 4 | 4 | 4 | 4 | 4 |
| 9 | Glyceryl 2-ethylhexanoate | 1 | 1 | 1 | 1 | 1 | 1 | 1 |
| 10 | Cyclomethicone | 20 | 20 | 20 | 20 | 20 | 20 | 20 |
| 11 | Copolymer-soluble matter of Synthesis Example 1 | 0.5 | 1 | 0.1 | - | - | - | - |
| 12 | Copolymer-soluble matter of Synthesis Comparative Example 1 | - | - | - | 0.5 | - | - | - |
| 13 | Copolymer-soluble matter of Synthesis Comparative Example 2 | - | - | - | - | 0.5 | - | - |
| 14 | Copolymer-soluble matter of Synthesis Comparative Example 3 | - | - | - | - | - | 0.5 | - |
| 15 | Copolymer-soluble matter of Synthesis Comparative Example 4 | - | - | - | - | - | - | 0.5 |
| 16 | Trimethylsiloxysilicic acid solution (Note 4) | 2 | 2 | 2 | 2 | 2 | 2 | 2 |
| 17 | Methylphenylpolysiloxane | 5 | 5 | 5 | 5 | 5 | 5 | 5 |
| 18 | Crosslinked methylpolysiloxane mixture (Note 5) | 2 | 2 | 2 | 2 | 2 | 2 | 2 |
| 19 | Crosslinked silicone-networked silicone block copolymer (Note 6) | 3 | 3 | 3 | 3 | 3 | 3 | 3 |
| 20 | Boron nitride | 1 | 1 | 1 | 1 | 1 | 1 | 1 |
| 21 | Ethyl paraoxybenzoate | 0.15 | 0.15 | 0.15 | 0.15 | 0.15 | 0.15 | 0.15 |
| 22 | Ethanol | 7 | 7 | 7 | 7 | 7 | 7 | 7 |
| 23 | 1,3-Butylene glycol | 3 | 3 | 3 | 3 | 3 | 3 | 3 |
| 24 | Phenylbenzimidazole sulfonic acid (Note 7) | 2.5 | 2.5 | 2.5 | 2.5 | 2.5 | 2.5 | 2.5 |
| 25 | Sodium chloride | 0.2 | 0.2 | 0.2 | 0.2 | 0.2 | 0.2 | 0.2 |
| 26 | Purified water | Balance | Balance | Balance | Balance | Balance | Balance | Balance |
| 27 | Perfume | q.s. | q.s. | q.s. | q.s. | q.s. | q.s. | q.s. |
| Evaluation item and judgement result | | | | | | | | |
| | Affinity to skin | Excellent | Excellent | Good | Fair | Good | Poor | Good |
| | Makeup-lasting effect | Excellent | Excellent | Good | Good | Poor | Good | Good |
| | Absence of sense of burden on skin | Excellent | Excellent | Good | Poor | Poor | Good | Poor |

| | | | | | | | | |
|---|---|---|---|---|---|---|---|---|
| (Note 1): OTS-2 TiO2 MP-1133 (manufactured by Daito Kasei Kogyo Co., Ltd.) (Note 2): KF-6028 (manufactured by Shin-Etsu Chemical Co., Ltd.) (Note 3): KF-6038 (manufactured by Shin-Etsu Chemical Co., Ltd.) (Note 4): KF-9021 (manufactured by Shin-Etsu Chemical Co., Ltd.) (Note 5): KSG-16 (manufactured by Shin-Etsu Chemical Co., Ltd.) (Note 6): KSP-100 (manufactured by Shin-Etsu Chemical Co., Ltd.) (Note 7): EUSOLEX 232 (manufactured by Merck Performance Materials Ltd.) | | | | | | | | |

In the present application, the term "copolymer-soluble matter" means a 1 mass% solution of the copolymer when the copolymer of any of Synthesis Examples 1 to 11 was used as the solute and a mixture of isopropyl alcohol and isododecane at a ratio of 1:2 was used as the solvent. For example, a "copolymer-soluble matter of Synthesis Example 1" means a 1 mass% solution of the copolymer of Synthesis Example 1 (solvent: a mixture of isopropyl alcohol and isododecane at a mass ratio of 1:2). The same applies to the "copolymer-soluble matters of Synthesis Examples 2 to 11".

Makeup bases of Copolymer Examples 1 to 3 and Copolymer Comparative Examples 1 to 4 were manufactured as follows:

### [Manufacturing method]

(1) Components 1 to 9 were treated with a three-roller mill;
(2) Components 10 to 15 were uniformly mixed;
(3) Components 16 and 17 and the components of (1) were uniformly dispersed in the mixture of (2);
(4) Components 18 to 27 were uniformly mixed; and
(5) The mixture of (4) was added to and emulsified in the mixture of (3), and defoaming was performed to prepare a W/O type makeup base.

### [Evaluation method]

Twenty cosmetic evaluation expert panelists (N = 20) individually evaluated the makeup bases of copolymer examples 1 to 3 and Copolymer Comparative Examples 1 to 4 for each of the items, "affinity to skin", "makeup-lasting effect", and "absence of sense of burden on skin", by 7-grade evaluation according to the following evaluation criteria, and the judgement was further made using the average score of all panelists according to the following judgement criteria:
[Evaluation criteria]
   (Evaluation result): (Score)
   Very good: 6 points
   Good: 5 points
   Slightly good: 4 points
   Fair: 3 points
   Slightly poor: 2 points
   Poor: 1 point
   Very poor: 0 points
[Judgement criteria]
   (Average score): (Judgement)
   5.0 or more: Excellent
   3.5 or more and less than 5.0: Good
   1.5 or more and less than 3.5: Fair
   Less than 1.5: Poor

It was proved from Table 3 that the W/O type bases of the present invention show very good effects in all of the "affinity to skin", "makeup-lasting effect", and "absence of sense of burden on skin" compared to the W/O type bases of Comparative Examples.

### [Manufacturing Examples 1 to 14 and Manufacturing Comparative Examples 1 to 9]

### (Manufacturing of treated powder)

The methacryloyloxyethyl phosphorylcholine-silicone methacrylate copolymer-treated powders of the present invention were manufactured using the methacryloyloxyethyl phosphorylcholine-silicone methacrylate copolymer manufactured in any of Synthesis Examples 1 to 11 as follows.

The methacryloyloxyethyl phosphorylcholine-silicone methacrylate copolymer manufactured by the method of any of Synthesis Examples 1 to 11 was dissolved in isopropyl alcohol as the solvent with a planetary mixer (stirring kneading machine), and a powder was then added thereto, followed by kneading in a form of slurry for 30 minutes. Incidentally, the manufacturing was performed at a mass ratio of the solvent and the methacryloyloxyethyl phosphorylcholine-silicone methacrylate copolymer and the powder, (solvent) : (methacryloyloxyethyl phosphorylcholine-silicone methacrylate copolymer and powder), of 1:2. Subsequently, the mixture was kneaded while removing the solvent in vacuum, and the resulting powder was taken out. The taken-out powder was then dried at 70°C for 10 hours and pulverized with a pulverizer to prepare each methacryloyloxyethyl phosphorylcholine-silicone methacrylate copolymer-treated powder. The treated powders according to Manufacturing Examples 1 to 14 were manufactured using the methacryloyloxyethyl phosphorylcholine-silicone methacrylate copolymers of the types and the amounts shown in Table 4.

Similarly, treated powders according to Manufacturing Comparative Examples 1 to 4 were manufactured using the methacryloyloxyethyl phosphorylcholine-silicone methacrylate copolymers manufactured by any of the above-described Synthesis Comparative Examples 1 to 4, and treated powders according to Manufacturing Comparative Examples 5 to 9 were manufactured using polymers other than the methacryloyloxyethyl phosphorylcholine-silicone methacrylate copolymers of Synthesis Comparative Examples 1 to 4.

### Evaluation of methacryloyloxyethyl phosphorylcholine-silicone methacrylate copolymer-treated powder

The methacryloyloxyethyl phosphorylcholine-silicone methacrylate copolymer-treated powders according to Manufacturing Examples 1 to 14 and Manufacturing Comparative Examples 1 to 4 and the polymer-treated powders according to Manufacturing Examples 5 to 9 were evaluated for the soft touch feeling (absence of sense of burden), skin adhesion, and spreadability. The evaluation items and results are shown in Table 4.

**[Table 4]**

| Manufacturing Example and Manufacturing Comparative Example | | Evaluation | | |
|---|---|---|---|---|
| | | Soft touch feeling (absence of sense of burden on skin) | Skin adhesion | Spreadability |
| Manufacturing Example 1 | Synthesis Example 1 copolymer (2.0%) treated talc | Excellent | Excellent | Excellent |
| Manufacturing Example 2 | Synthesis Example 1 copolymer (0.1%) treated talc | Excellent | Good | Excellent |
| Manufacturing Example 3 | Synthesis Example 1 copolymer (10%) treated talc | Excellent | Excellent | Good |
| Manufacturing Example 4 | Synthesis Example 1 copolymer (10%) treated talc | Excellent | Excellent | Excellent |
| Manufacturing Example 5 | Synthesis Example 2 copolymer (2%) treated talc | Excellent | Excellent | Excellent |
| Manufacturing Example 6 | Synthesis Example 3 copolymer (2%) treated talc | Excellent | Excellent | Excellent |
| Manufacturing Example 7 | Synthesis Example 4 copolymer (2%) treated talc | Excellent | Excellent | Excellent |
| Manufacturing Example 8 | Synthesis Example 5 copolymer (2%) treated talc | Excellent | Excellent | Excellent |
| Manufacturing Example 9 | Synthesis Example 6 copolymer (2%) treated talc | Excellent | Excellent | Excellent |
| Manufacturing Example 10 | Synthesis Example 7 copolymer (2%) treated talc | Excellent | Excellent | Excellent |
| Manufacturing Example 11 | Synthesis Example 8 copolymer (2%) treated talc | Excellent | Excellent | Excellent |
| Manufacturing Example 12 | Synthesis Example 9 copolymer (2%) treated talc | Excellent | Excellent | Excellent |
| Manufacturing Example 13 | Synthesis Example 10 copolymer (2%) treated talc | Excellent | Excellent | Excellent |
| Manufacturing Example 14 | Synthesis Example 11 copolymer (2%) treated talc | Excellent | Excellent | Excellent |
| Manufacturing Comparative Example 1 | Synthesis Comparative Example 1 copolymer (2%) treated talc | Poor | Excellent | Excellent |
| Manufacturing Comparative Example 2 | Synthesis Comparative Example 2 copolymer (2%) treated talc | Excellent | Excellent | Poor |
| Manufacturing Comparative Example 3 | Synthesis Comparative Example 3 copolymer (2%) treated talc | Poor | Poor | Good |
| Manufacturing Comparative Example 4 | Synthesis Comparative Example 4 copolymer (2%) treated talc | Poor | Excellent | Excellent |
| Manufacturing Comparative Example 5 | Triethoxycaprylylsilane (2.0%) treated talc | Poor | Good | Poor |
| Manufacturing Comparative Example 6 | Methyl hydrogen polysiloxane (2.0%) treated talc | Poor | Poor | Good |
| Manufacturing Comparative Example 7 | Silicone (2.0%) treated talc | Poor | Fair | Good |
| Manufacturing Comparative Example 8 | Silicone acrylate (2.0%) treated talc | Poor | Good | Good |
| Manufacturing Comparative Example 9 | Polymethacryloyloxyethyl phosphorylcholine (2.0%) treated talc | Good | Good | Fair |
| The talc used was Talc JA-46R (manufactured by Asada Milling Co., Ltd.) | | | | |

### [Treated powder example]

### Face powder (powder form)

The cosmetic film uniformity, makeup-lasting effect, and soft sense of use of face powders (powder form) including the methacryloyloxyethyl phosphorylcholine-silicone methacrylate copolymer-treated powders according to Manufacturing Examples 1 and 2 were evaluated. The evaluation methods and results are shown in Table 5.

**[Table 5]**

| Methacryloyloxyethyl phosphorylcholine-silicone methacrylate copolymer surface treatment agent | | | | | | | | | | |
|---|---|---|---|---|---|---|---|---|---|---|
| Face powder (powder form) | | | | | | | | | | |
| (%) | | | | | | | | | | |
| No. | Component | Powder Treatment Example | | | | Powder Treatment Comparative Example | | | | |
| | | 1 | 2 | 3 | 4 | 1 | 2 | 3 | 4 | 5 |
| 1 | Manufacturing Example 1 (2.0%) treated titanium oxide | 10 | - | - | - | - | - | - | - | - |
| 2 | Manufacturing Example 2 (2.0%) treated titanium oxide | - | 10 | - | - | - | - | - | - | - |
| 3 | Manufacturing Example 1 (0.1%) treated titanium oxide | - | - | 10 | - | - | - | - | - | - |
| 4 | Manufacturing Example 1 (10%) treated titanium oxide | - | - | - | 10 | - | - | - | - | - |
| 5 | Triethoxycaprylylsilane (2.0%) treated titanium oxide (Note 1) | - | - | - | - | 10 | - | - | - | - |
| 6 | Methyl hydrogen polysiloxane (2.0%) treated titanium oxide (Note 2) | - | - | - | - | - | 10 | - | - | - |
| 7 | Silicone (2.0%) treated titanium oxide (Note 3) | - | - | - | - | - | - | 10 | - | - |
| 8 | Silicone acrylate (2.0%) treated titanium oxide (Note 4) | - | - | - | - | - | - | - | 10 | - |
| 9 | Polymethacryloyloxyethyl phosphorylcholine (2.0%) treated titanium oxide (Note 5) | - | - | - | - | - | - | - | - | 10 |
| 10 | Red iron oxide | 0.1 | 0.1 | 0.1 | 0.1 | 0.1 | 0.1 | 0.1 | 0.1 | 0.1 |
| 11 | Yellow iron oxide | 0.2 | 0.2 | 0.2 | 0.2 | 0.2 | 0.2 | 0.2 | 0.2 | 0.2 |
| 12 | Black iron oxide | 0.05 | 0.05 | 0.05 | 0.05 | 0.05 | 0.05 | 0.05 | 0.05 | 0.05 |
| 13 | Talc | Balance | Balance | Balance | Balance | Balance | Balance | Balance | Balance | Balance |
| 14 | Mica | 10 | 10 | 10 | 10 | 10 | 10 | 10 | 10 | 10 |
| 15 | Synthetic phlogopite | 10 | 10 | 10 | 10 | 10 | 10 | 10 | 10 | 10 |
| 16 | Nylon powder | 10 | 10 | 10 | 10 | 10 | 10 | 10 | 10 | 10 |
| 17 | Crosslinked silicone-networked silicone block copolymer | 10 | 10 | 10 | 10 | 10 | 10 | 10 | 10 | 10 |
| 18 | Boron nitride | 2 | 2 | 2 | 2 | 2 | 2 | 2 | 2 | 2 |
| 19 | Preservative | q.s. | q.s. | q.s. | q.s. | q.s. | q.s. | q.s. | q.s. | q.s. |
| 21 | Glyceryl 2-ethylhexanoate | 0.02 | 0.02 | 0.02 | 0.02 | 0.02 | 0.02 | 0.02 | 0.02 | 0.02 |
| 22 | Dimethicone | 0.02 | 0.02 | 0.02 | 0.02 | 0.02 | 0.02 | 0.02 | 0.02 | 0.02 |
| 23 | Liquid paraffin | 0.02 | 0.02 | 0.02 | 0.02 | 0.02 | 0.02 | 0.02 | 0.02 | 0.02 |
| 24 | Perfume | q.s. | q.s. | q.s. | q.s. | q.s. | q.s. | q.s. | q.s. | q.s. |
| Evaluation item and judgement result | | | | | | | | | | |
| | Cosmetic film uniformity | Excellent | Excellent | Good | Excellent | Fair | Poor | Fair | Fair | Good |
| | Makeup-lasting effect | Excellent | Excellent | Good | Excellent | Poor | Poor | Poor | Good | Poor |
| | Soft sense of use | Excellent | Excellent | Excellent | Good | Fair | Poor | Fair | Poor | Good |

| | | | | | | | | | | |
|---|---|---|---|---|---|---|---|---|---|---|
| Note 1: OTS-treated CR-50 (manufactured by Daito Kasei Kogyo Co., Ltd.) Note 2: 99P treatment (manufactured by Shin-Etsu Chemical Co., Ltd.) Note 3: SA-treated CR-50 (manufactured by Miyoshi Kasei, Inc.) Note 4: KP-540 treatment (manufactured by Shin-Etsu Chemical Co., Ltd.) Note 5: Lipidure HM-600 treatment (manufactured by NOF Corporation) | | | | | | | | | | |

### [Manufacturing method]

(1) Components 1 to 19 were uniformly mixed;
(2) Components 21 to 24 were uniformly mixed;
(3) The mixture of (2) was added to and uniformly mixed with the mixture of (1); and
(4) The mixture of (3) was pulverized and put in a container to prepare a face powder (powder form).

The evaluation of cosmetic film uniformity, makeup-lasting effect, and soft sense of use in Table 3 is sensory evaluation by expert panelists (N = 20).

### [Evaluation method]

Twenty cosmetic evaluation expert panelists (N = 20) individually evaluated the face powders (powder form) of Powder Treatment Examples 1 to 4 and Powder Treatment Comparative Examples 1 to 5 for each of the items, "cosmetic film uniformity", "makeup-lasting effect", and "soft sense of use (absence of sense of burden on skin)", by 7-grade evaluation according to the following evaluation criteria, and the judgement was further made using the average score of all panelists according to the following judgement criteria:
[Evaluation criteria]
   (Evaluation result): (Score)
   Very good: 6 points
   Good: 5 points
   Slightly good: 4 points
   Fair: 3 points
   Slightly poor: 2 points
   Poor: 1 point
   Very poor: 0 points
[Judgement criteria]
   (Average score): (Judgement)
   5.0 or more: Excellent
   3.5 or more and less than 5.0: Good
   1.5 or more and less than 3.5: Fair
   Less than 1.5: Poor

Examples for from skin care formulations to makeup formulations including copolymer-soluble matters of the present invention manufactured by Synthesis Examples above will be described below for each dosage form.

### Example 1: Emulsion type lotion

### (Component): (%)

1. Cetyl alcohol: 0.2
2. Copolymer-soluble matter of Synthesis Example 1: 0.2
3. Mixture of phospholipid and phytosterol (15:85) (Note 1): 0.5
4. Polyoxyethylene (40 mol) hydrogenated castor oil: 0.1
5. Tocopherol acetate: 0.5
6. Triethanolamine stearate: 0.5
7. Purified water: balance
8. Ethanol: 10.0
9. Perfume: q.s.
(Note 1): manufactured by Nippon Fine Chemical Co., Ltd.

### (Manufacturing method)

(1) Components 1 to 6 were heated to 75°C and were uniformly mixed and dissolved;
(2) Components 7 and 8 were heated to 75°C and were uniformly mixed and dissolved;
(3) The mixture of (2) was added to the mixture of (1), followed by emulsification; and
(4) The emulsion of (3) was cooled, and component 9 was added thereto to prepare an emulsion type lotion.

The emulsion type lotion of Example 1 was excellent in "skin affinity", "makeup-lasting effect", and "absence of sense of burden on skin".

### Example 2: Emulsion

### (Component): (%)

1. Stearic acid: 0.5
2. Polyoxyethylene sorbitan monostearate (20 E.O.): 1.0
3. Polyoxypropylene sorbitol tetraoleate (40 E.O.): 1.0
4. Behenyl alcohol: 1.5
4. Behenyl alcohol: 1.5
5. Liquid paraffin: 2.0
6. Glyceryl tri(2-ethylhexanoate): 5.0
7. Copolymer-soluble matter of Synthesis Example 2: 2.0
8. Acrylic acid-methacrylic acid alkyl ester copolymer: 0.1
9. Xanthan gum: 0.1
10. Sodium hydroxide: 0.05
11. 1,3-Butylene glycol: 8.0
12. Preservative: q.s.
13. Perfume: q.s.
14. Purified water: balance

### (Manufacturing method)

(1) Components 1 to 7 were uniformly mixed at 80°C;
(2) Components 8 to 14 were uniformly mixed at 80°C;
(3) The mixture of (2) was added to the mixture of (1), followed by emulsification; and
(4) The emulsion of (3) was cooled while stirring to prepare an emulsion.

The emulsion of Example 2 was excellent in "skin affinity", "makeup-lasting effect", and "absence of sense of burden on skin".

### Example 3: O/W type cream

### (Component): (%)

1. Decaglyceryl pentaoleate: 2.5
2. Behenyl alcohol: 1.5
3. Vaseline: 3.0
4. Heavy liquid isoparaffin: 1.0
5. Glyceryl tri(2-ethylhexanoate): 1.0
6. Copolymer-soluble matter of Synthesis Example 3: 2.0
7. Purified water: balance
8. Glycerin: 7.0
9. Acrylic acid-methacrylic acid alkyl ester copolymer: 0.2
10. Sodium hydroxide: 0.09
11. Preservative: q.s.
12. Perfume: q.s.

### (Manufacturing method)

(1) Components 1 to 6 were uniformly mixed at 80°C;
(2) Components 7 to 12 were uniformly mixed at 80°C;
(3) The mixture of (2) was added to the mixture of (1), followed by emulsification; and
(4) The emulsion of (3) was cooled while stirring to prepare a cream.

The O/W type cream of Example 3 was excellent in "skin affinity", "makeup-lasting effect", and "absence of sense of burden on skin".

### Example 4: W/O type cream

### (Component): (%)

1. Microcrystalline wax: 1.5
2. Bees wax: 1.5
3. Cetyl alcohol: 3.0
4. Copolymer-soluble matter of Synthesis Example 4: 2.0
5. Hydrogenated soybean phospholipid: 1.0
6. Squalane: 35.0
7. Sorbitan sesquioleate: 3.0
8. Propylene glycol: 10.0
9. Preservative: q.s.
10. Perfume: q.s.
11. Purified water: balance

### (Manufacturing method)

(1) Components 1 to 7 were heated to 75°C and were uniformly mixed and dissolved;
(2) Components 8 and 11 were heated to 75°C and were uniformly mixed and dissolved;
(3) The mixture of (2) was added to the mixture of (1), followed by emulsification; and
(4) The emulsion of (3) was cooled, and components 9 and 10 were added thereto to prepare a W/O type cream.

The W/O type cream of Example 4 was excellent in "skin affinity", "makeup-lasting effect", and "absence of sense of burden on skin".

### Example 5: Oil cleanser

### (Component): (%)

1. Polyoxyethylene sorbitol tetraoleate: 8.0
2. Polyglyceryl diisostearate: 0.5
3. Liquid paraffin: balance
4. Glyceryl tri(2-ethylhexanoate): 25.0
5. Copolymer-soluble matter of Synthesis Example 5: 1.0
6. Preservative: q.s.
7. Perfume: q.s.

### (Manufacturing method)

Components 1 to 7 were uniformly mixed at ordinary temperature to prepare an oil cleanser.

The oil cleanser of Example 5 was excellent in "skin affinity" and "absence of sense of burden on skin".

### Example 6: Cleansing cream

### (Component): (%)

1. Stearic acid: 3.0
2. Cetanol: 2.0
3. Polyoxypropylene sorbitol tetraoleate (40 E.O.): 1.0
4. Polyoxyethylene sorbitan monostearate (20 E.O.): 1.0
5. Glyceryl tri(2-ethylhexanoate): 20.0
6. Liquid paraffin: 20.0
7. Copolymer-soluble matter of Synthesis Example 6: 2.0
8. Acrylic acid-methacrylic acid alkyl ester copolymer: 0.1
9. 1,3-Butylene glycol: 7.0
10. Sodium hydroxide: 0.05
11. Purified water: balance
12. Preservative: q.s.
13. Perfume: q.s.

### (Manufacturing method)

(1) Components 1 to 7 were uniformly mixed at 80°C;
(2) Components 8 to 13 were uniformly mixed at 80°C;
(3) The mixture of (1) was added to the mixture of (2), followed by emulsification; and
(4) The emulsion of (3) was cooled while stirring to prepare a cleansing cream.

The cleansing cream of Example 6 was excellent in "skin affinity" and "absence of sense of burden on skin".

### Example 7: Styling water

### (Component): (%)

1. Ethanol: 15.0
2. Stearyl trimethylammonium chloride: 0.2
3. Polyoxyethylene isostearate hydrogenated castor oil: 0.2
4. Copolymer-soluble matter of Synthesis Example 7: 0.1
5. Purified water: balance
6. Hydroxypropyl cellulose: 0.01
7. Highly polymerized methylpolysiloxane emulsion (Note 1): 2.0
8. Preservative: q.s.
9. Perfume: q.s.

(Note 1) BY11-007 (manufactured by Dow Toray Co., Ltd.)

### (Manufacturing method)

Components 1 to 9 were uniformly mixed at ordinary temperature to prepare a styling water.

The styling water of Example 7 was excellent in "affinity to hair", "makeup-lasting effect", and "absence of sense of burden on hair".

### Example 8: Hair wax

### (Component): (%)

1. Purified water: balance
2. Propylene glycol: 10.0
3. Polyethylene glycol monostearate: 3.0
4. Vaseline: 10.0
5. Paraffin wax: 3.0
6. Cetostearyl alcohol: 3.0
7. Behenyl alcohol: 3.0
8. Copolymer-soluble matter of Synthesis Example 8: 2.0
9. Ethanol: 5.0
10. Purified water: 15.0
11. Vinylpyrrolidone: 1.0
12. Acrylic acid-methacrylic acid alkyl ester copolymer: 0.15
13. Sodium hydroxide: 0.05
14. Preservative: q.s.
15. Perfume: q.s.

### (Manufacturing method)

(1) Components 1 to 3 were uniformly mixed at 80°C;
(2) Components 4 to 8 were uniformly mixed at 80°C;
(3) The mixture of (2) was added to the mixture of (1), followed by emulsification; and
(4) Components 9 to 15 were added to the emulsion of (3), followed by cooling while stirring to prepare a hair wax.

The hair wax of Example 8 was excellent in "affinity to hair", "makeup-lasting effect", and "absence of sense of burden on hair".

### Example 9: O/W type mascara

### (Component): (%)

1. Stearic acid: 2.0
2. Bees wax: 10.0
3. Cetostearyl alcohol: 1.0
4. Polyoxyethylene sorbitan monooleate (20 E.O.): 1.5
5. Sorbitan sesquioleate: 0.5
6. Copolymer-soluble matter of Synthesis Example 9: 3.0
7. Black iron oxide: 5.0
8. Silicic anhydride: 3.0
9. Purified water: balance
10. 1,3-Butylene glycol: 10.0
11. Triethanolamine: 1.5
12. Alkyl acrylate copolymer emulsion (Note 1): 30.0
13. Preservative: q.s.
14. Perfume: q.s.

(Note 1) Yodosol 32A707 (solid content: 45%) (manufactured by Nippon NSC Ltd.)

### (Manufacturing method)

(1) Components 1 to 3 were uniformly mixed at 80°C;
(2) Components 4 to 8 were treated with a roller;
(3) Components 9 to 14 were uniformly mixed at 80°C;
(4) The mixture of (1) and the components of (2) were mixed with each other, and the mixture of (3) was added thereto, followed by emulsification; and
(5) The emulsion of (4) was cooled to prepare an O/W type mascara.

The O/W type mascara of Example 9 was excellent in "affinity to eyelashes", "makeup-lasting effect", and "absence of sense of burden on eyelashes".

### Example 10: Paste lip gloss

### (Component): (%)

1. Copolymer-soluble matter of Synthesis Example 1: 2.0
2. Diisostearyl malate: 10.0
3. Stearyl lactate: 10.0
4. Hydrogenated polyisobutene: 30.0
5. Triethylhexanoin: balance
6. Liquid paraffin: 5.0
7. Microcrystalline wax: 2.0
8. 12-Hydroxystearic acid: 0.1
9. α-Olefin-vinylpyrrolidone copolymer (Note 1): 0.5
10. Silicic anhydride (Note 2): 3.5
11. Red iron oxide: 0.1
12. Red No. 201: 0.3
13. Black iron oxide: 0.05
14. Titanium oxide: 0.2
15. Soybean phospholipid: 0.01
16. 2-Ethylhexyl p-methoxycinnamate: 3.0
17. Preservative: q.s.
18. Perfume: q.s.

(Note 1) ANTARON V-220 (manufactured by ISP Co., Ltd.)
(Note 2) AEROSIL 200 (manufactured by Nippon Aerosil Co., Ltd.)

### (Manufacturing method)

(1) Components 1 to 9 were dissolved and mixed at 100°C;
(2) Components 10 to 18 were added to the mixture of (1), followed by uniform mixing and dispersion; and
(3) The dispersion of (2) was flown into a tube and cooled to prepare a paste lip gloss.

The paste lip gloss of Example 10 was excellent in "affinity to lips", "makeup-lasting effect", and "absence of sense of burden on lips".

### Example 11: Stick rouge

### (Component): (%)

1. Polyethylene wax: 10.0
2. Carnauba wax: 5.0
3. Paraffin wax: 2.0
4. Cetyl 2-ethylhexanoate: balance
5. Copolymer-soluble matter of Synthesis Example 2: 2.0
6. Liquid paraffin: 10.0
7. Isotridecyl isononanate: 10.0
8. Red No. 202: 0.5
9. Yellow No. 4: 2.0
10. Titanium oxide: 0.5
11. Black iron oxide: 0.1
12. α-Tocopherol: 0.5
13. Perfume: q.s.

### (Manufacturing method)

(1) Components 1 to 7 were uniformly dissolved and mixed at 100°C;
(2) Components 8 to 13 were added to and uniformly mixed with the mixture of (1); and
(3) The mixture of (2) was flown into a container and cooled to prepare a stick rouge.

The stick rouge of Example 11 was excellent in "affinity to lips", "makeup-lasting effect", and "absence of sense of burden on lips".

### Example 12: Oil eye shadow

### (Component): (%)

1. Dextrin palmitate (Note 1): 2.0
2. Copolymer-soluble matter of Synthesis Example 3: 1.0
3. Diisostearyl malate: 2.5
4. Cetyl 2-ethylhexanoate: 13.0
5. Dextrin laurate: 2.0
6. Dextrin myristate: 5.0
7. Dextrin behenate: 5.0
8. Liquid paraffin: balance
9. Hydrogenated polyisobutene: 1.5
10. Silicic anhydride: 6.0
11. Nylon powder: 5.0
12. Silicone-treated talc (Note 2): 5.5
13. Red No. 202: 0.05
14. Yellow No. 4 aluminum lake: 0.05
15. Blue No. 1 aluminum lake: 0.05
16. Titanated mica: 1.5
17. Preservative: q.s.
18. Perfume: q.s.

(Note 1) Rheopearl TT (manufactured by Chiba Flour Milling Co., Ltd.)
(Note 2) Treated with 5% dimethylpolysiloxane

### (Manufacturing method)

(1) Components 1 to 9 were dissolved and mixed at 100°C;
(2) Components 10 to 18 were added to the mixture of (1), followed by uniform mixing and dispersion; and
(3) The dispersion of (2) was flown into a container and cooled and solidified to prepare an oil eye color.

The oil eye shadow of Example 12 was excellent in "affinity to eyelids", "makeup-lasting effect", and "absence of sense of burden on eyelids".

### Example 13: Solid powder foundation

### (Component): (%)

1. Dimethylpolysiloxane-treated talc: 30.0
2. Dimethylpolysiloxane-treated mica: 15.0
3. Dimethylpolysiloxane-treated titanium oxide: 15.0
4. Dimethylpolysiloxane-treated sericite: balance
5. Synthetic phlogopite: 5.0
6. Yellow iron oxide: 2.0
7. Red iron oxide: 0.5
8. Black iron oxide: 0.2
9. Preservative: q.s.
10. Copolymer-soluble matter of Synthesis Example 4: 1.0
11. Liquid paraffin: 3.0
12. Dimethylpolysiloxane: 1.0
13. Glyceryl 2-ethylhexanoate: 2.0
14. Perfume: q.s.

### (Manufacturing method)

(1) Components 1 to 9 were uniformly dispersed at 75°C with a Henschel mixer (manufactured by Mitsui Miike Machinery Co., Ltd.);
(2) Components 10 to 13 were uniformly mixed and dissolved;
(3) The mixture of (2) and component 14 were added to the dispersion of (1) while stirring with the Henschel mixer to uniformly disperse them;
(4) The dispersion of (3) was pulverized with a pulverizer; and
(5) The powder of (4) was put in a metal dish and pressure-molded to prepare a solid powder foundation.

The solid powder foundation of Example 13 was excellent in "affinity to skin", "makeup-lasting effect", and "absence of sense of burden on skin".

### Example 14: Solid powder eye shadow

### (Component): (%)

1. Dimethylpolysiloxane-treated synthetic gold mica: 10.0
2. Dimethylpolysiloxane-treated talc: balance
3. Titanium oxide-coated mica: 30.0
4. Boron nitride: 5.0
5. Polyethylene terephthalate-aluminum-epoxy laminated powder: 5.0
6. Ultramarine blue: 2.0
7. Red No. 202: 0.5
8. Alkyl polyacrylate: 1.0
9. Preservative: q.s.
10. Copolymer-soluble matter of Synthesis Example 5: 1.0
11. Liquid paraffin: 3.0
12. Dimethylpolysiloxane: 2.0
13. Glyceryl 2-ethylhexanoate: 2.0
14. Perfume: q.s.

### (Manufacturing method)

(1) Components 1 to 9 were uniformly dispersed at 75°C with a Henschel mixer (manufactured by Mitsui Miike Machinery Co., Ltd.);
(2) Components 10 to 13 were uniformly mixed and dissolved;
(3) The mixture of (2) and component 14 were added to the dispersion of (1) while stirring with the Henschel mixer to uniformly disperse them;
(4) The dispersion of (3) was pulverized with a pulverizer; and
(5) The powder of (4) was put in a metal dish and pressure-molded to prepare a solid powder eye shadow.

The solid powder eye shadow of Example 14 was excellent in "affinity to eyelids", "makeup-lasting effect", and "absence of sense of burden on eyelids".

### Example 15: Solid powder face color

### (Component): (%)

1. Mica: 20.0
2. Talc: balance
3. Titanium oxide-coated mica: 10.0
4. Ultramarine blue: 0.5
5. Red No. 226: 0.2
6. Alkyl polyacrylate: 1.0
7. Preservative: q.s.
8. Copolymer-soluble matter of Synthesis Example 6: 1.0
9. Liquid paraffin: 2.0
10. Dimethylpolysiloxane: 1.0
11. Glyceryl 2-ethylhexanoate: 1.0
12. Perfume: q.s.

### (Manufacturing method)

(1) Components 1 to 7 were uniformly dispersed at 75°C with a Henschel mixer (manufactured by Mitsui Miike Machinery Co., Ltd.);
(2) Components 8 to 11 were heated to 65°C and uniformly mixed and dissolved;
(3) The mixture of (2) and component 12 were added to the dispersion of (1) while stirring with the Henschel mixer to uniformly disperse them;
(4) The dispersion of (3) was pulverized with a pulverizer; and
(5) The powder of (4) was put in a metal dish and pressure-molded to prepare a solid powder face color.

The solid powder face color of Example 15 was excellent in "affinity to skin", "makeup-lasting effect", and "absence of sense of burden on skin".

### Example 16: Stick concealer

### (Component): (%)

1. Paraffin wax: 5.0
2. Polyethylene wax: 5.0
3. Candelilla wax: 2.0
4. Copolymer-soluble matter of Synthesis Example 7: 2.0
5. Glyceryl tri(2-ethylhexanoate): balance
6. Methylphenylpolysiloxane: 5.0
7. Liquid lanolin acetate: 10.0
8. 2-Ethylhexyl paramethoxycinnamate: 5.0
9. Titanium oxide: 20.0
10. Yellow iron oxide: 2.0
11. Red iron oxide: 0.5
12. Black iron oxide: 0.2
13. Mica: 7.0
14. Preservative: q.s.

### (Manufacturing method)

(1) Components 1 to 8 were mixed and dissolved at 100°C;
(2) Components 9 to 14 were uniformly mixed with the mixture of (1) at 90°C;
(3) The mixture of (2) was treated with a three-roller mill; and
(4) The mixture of (3) was defoamed and was dissolved and put in a capsule at 85°C and was then cooled at 4°C to prepare a stick concealer.

The stick concealer of Example 16 was excellent in "affinity to skin", "makeup-lasting effect", and "absence of sense of burden on skin".

### Example 17: W/O type sunscreen

### (Component): (%)

1. Zinc oxide: 2.0
2. Silicone-coated fine titanium oxide: 5.0
3. Caprylic/capric triglyceride: 5.0
4. Glyceryl tri(2-ethylhexanoate): 3.0
5. Octyl palmitate: 3.0
6. 2-Ethylhexyl paramethoxycinnamate: 10.0
7. Decamethylcyclopentasiloxane: 10.0
8. Methylpolysiloxane-cetylmethylpolysiloxane-poly(oxyethylene/oxypropylene)methylpolysiloxane copolymer (Note 1): 1.8
9. Copolymer-soluble matter of Synthesis Example 8: 1.0
10. Preservative: q.s.
11. Sodium chloride: 0.3
12. Purified water: balance
13. Dipropylene glycol: 5.0
14. Ethanol: 5.0
15. Perfume: q.s.

(Note 1) ABIL EM-90 (manufactured by Evonik Goldschmidt GmbH)

### (Manufacturing method)

(1) Components 3 and 4 were heated and dissolved, and components 1 and 2 were then added thereto, followed by uniform dispersion with a roller;
(2) Components 5 to 10 were dissolved at 70°C, and the dispersion of (1) was added thereto at 60°C, followed by uniform mixing and dissolving;
(3) Components 11 to 13 were mixed and dissolved and then added to the mixture of (2) at 60°C, followed by emulsification; and
(4) Components 14 and 15 were added to and uniformly mixed with the emulsion of (3) to prepare a W/O type sunscreen.

The W/O type sunscreen of Example 17 was excellent in "affinity to skin", "makeup-lasting effect", and "absence of sense of burden on skin".

### Example 18: W/O type foundation

### (Component): (%)

1. Polyoxyethylenemethylsiloxane-polyoxypropyleneoleylmethylsiloxane-dimethylsiloxane copolymer (Note 1): 2.0
2. PEG-3 dimethicone (Note 2): 1.0
3. Cyclopentasiloxane: 20.0
4. Silicone-treated red iron oxide: 1.0
5. Silicone-treated yellow iron oxide: 1.5
6. Silicone-treated black iron oxide: 0.5
7. Silicone-treated titanium oxide: 10.0
8. Silicone-treated talc: 5.0
9. Glyceryl tri(2-ethylhexanoate): 5.0
10. Copolymer-soluble matter of Synthesis Example 9: 1.0
11. Sorbitan sesquioleate: 0.5
12. Purified water: balance
13. 1,3-Butylene glycol: 15.0
14. Sodium chloride: 0.5
15. Preservative: q.s.
16. Perfume: q.s.

(Note 1) KF-6026 (manufactured by Shin-Etsu Chemical Co., Ltd.)
(Note 2) KF-6015 (manufactured by Shin-Etsu Chemical Co., Ltd.)

### (Manufacturing method)

(1) Components 1 to 3 were uniformly mixed;
(2) Components 4 to 11 were uniformly dispersed with a roller;
(3) The dispersion of (2) was added to and uniformly mixed with the mixture of (1); and
(4) Components 12 to 16 were added to the mixture of (3), followed by emulsification to prepare a W/O type foundation.

The W/O type foundation of Example 18 was excellent in "affinity to skin", "makeup-lasting effect", and "absence of sense of burden on skin".

### Example 19: O/W type foundation

### (Component): (%)

1. Polyoxyethylene sorbitan monooleate (20 E.O.): 0.5
2. Sorbitan sesquioleate: 0.5
3. 1,3-Butylene glycol: 10.0
4. Silicone-treated titanium oxide: 10.0
5. Silicone-treated red iron oxide: 0.4
6. Silicone-treated yellow iron oxide: 2.0
7. Silicone-treated black iron oxide: 0.1
8. Silicone-treated talc: 5.0
9. Carboxyvinyl polymer: 0.3
10. Triethanolamine: 1.0
11. Purified water: balance
12. Ethanol: 2.0
13. Stearic acid: 1.0
14. Behenyl alcohol: 0.5
15. Liquid paraffin: 5.0
16. Copolymer-soluble matter of Synthesis Example 1: 1.0
17. Glyceryl tri(2-ethylhexanoate): 1.0
18. 2-Ethylhexyl paramethoxycinnamate: 2.0
19. Vaseline: 0.5
20. Preservative: q.s.
21. Perfume: q.s.

### (Manufacturing method)

(1) Components 1 to 8 were uniformly dispersed with a roller;
(2) Components 9 to 12 were uniformly mixed;
(3) The dispersion of (1) was added to and uniformly mixed with the mixture of (2);
(4) Components 13 to 20 were mixed and dissolved at 80°C;
(5) The mixture of (4) was added to the mixture of (3) at 80°C, followed by emulsification; and
(6) The emulsion of (5) was cooled, and component 21 was added thereto to prepare an O/W type foundation.

The O/W type foundation of Example 19 was excellent in "affinity to skin", "makeup-lasting effect", and "absence of sense of burden on skin".

### Example 20: Oil solid foundation

### (Component): (%)

1. Talc: 15.0
2. Mica: 10.0
3. Silicone-treated titanium oxide: 15.0
4. Silicone-treated red iron oxide: 1.0
5. Silicone-treated yellow iron oxide: 3.0
6. Silicone-treated black iron oxide: 0.2
7. Polyethylene wax: 7.0
8. Microcrystalline wax: 6.0
9. Glyceryl tri(2-ethylhexanoate): balance
10. Copolymer-soluble matter of Synthesis Example 2: 3.0
11. Dimethylpolysiloxane: 5.0
12. Liquid paraffin: 20.0
13. Polyoxyethylenemethylsiloxane-polyoxypropyleneoleylmethylsiloxane-dimethylsiloxane copolymer (Note 1): 2.0
14. Preservative: q.s.
15. Perfume: q.s.

(Note 1) KF-6026 (manufactured by Shin-Etsu Chemical Co., Ltd.)

### (Manufacturing method)

(1) Components 7 to 14 were heated and dissolved at 90°C;
(2) Components 1 to 6 were added to the solution of (1) and were uniformly dispersed with a roller; and
(3) Component 15 was added to the dispersion of (2) and dissolved at 80°C, and the solution was then put in a metal dish to prepare an oil solid foundation.

The oil solid foundation of Example 20 was excellent in "affinity to skin", "makeup-lasting effect", and "absence of sense of burden on skin".

### Example 21: O/W type makeup base

### (Component): (%)

1. Cetostearyl alcohol: 2.0
2. 2-Ethylhexyl paramethoxycinnamate: 5.0
3. Copolymer-soluble matter of Synthesis Example 3: 2.0
4. Glyceryl tri(2-ethylhexanoate): 3.0
5. Purified water: balance
6. N-Stearoyl-N-methyltaurine sodium: 0.5
7. Carboxyvinyl polymer: 0.1
8. Acrylic acid-methacrylic acid alkyl ester copolymer: 0.1
9. Sodium hydroxide: 0.05
10. Ethanol: 10.0
11. 1,3-Butylene glycol: 10.0
12. Preservative: q.s.
13. Perfume: q.s.

### (Manufacturing method)

(1) Components 1 to 4 were uniformly dissolved at 80°C;
(2) Components 5 to 12 were uniformly dissolved at 80°C;
(3) The solution of (1) was added to the solution of (2), followed by emulsification; and
(4) Component 13 was added to the emulsion of (3), followed by cooling to prepare a makeup base.

The O/W type makeup base of Example 21 was excellent in "affinity to skin", "makeup-lasting effect", and "absence of sense of burden on skin".

### Example 22: Body milk

### (Component): (%)

1. Stearic acid: 1.0
2. Polyoxyethylene sorbitan monooleate (20 E.O.): 0.5
3. Sorbitan sesquioleate: 0.5
4. Behenyl alcohol: 0.5
5. Glyceryl 2-ethylhexanoate: 2.0
6. Liquid paraffin: 2.0
7. Copolymer-soluble matter of Synthesis Example 4: 2.0
8. Ethanol: 10.0
9. Dipropylene glycol: 10.0
10. Triethanolamine: 1.0
11. Purified water: balance
12. Glycerin: 5.0
13. 1,3-Butylene glycol: 5.0
14. Acrylic acid-methacrylic acid alkyl ester copolymer: 0.2
15. Preservative: q.s.
16. Perfume: q.s.

### (Manufacturing method)

(1) Components 1 to 7 were uniformly dissolved at 80°C;
(2) Components 8 to 16 were uniformly dissolved at 80°C;
(3) The solution of (1) was added to the solution of (2), followed by emulsification; and
(4) The emulsion of (3) was stirred and cooled to prepare a body milk.

The body milk of Example 22 was excellent in "affinity to skin", "makeup-lasting effect", and "absence of sense of burden on skin".

### Example 23: Makeup remover

### (Component): (%)

1. 1,3-Butylene glycol: 15.0
2. Alkyl acrylate copolymer emulsion (Note 1): 2.5
3. L-Arginine: 0.5
4. Purified water: balance
5. Light liquid isoparaffin: 3.0
6. Copolymer-soluble matter of Synthesis Example 5: 0.2
7. Preservative: q.s.
8. Perfume: q.s.

(Note 1) Yodosol 32A707 (solid content: 45%) (manufactured by Nippon NSC Ltd.)

### (Manufacturing method)

(1) Components 1 to 4 were uniformly mixed;
(2) Components 5 to 8 were added to the mixture of (1) at 60°C, followed by emulsification; and
(3) The emulsion of (2) was cooled while stirring to prepare a makeup remover.

The makeup remover of Example 23 was excellent in "affinity to skin" and "absence of sense of burden on skin".

### Example 24: Oil mascara

### (Component): (%)

1. Pentaerythrityl rosinate: 10.0
2. Candekilla resin: 3.0
3. Bees wax: 2.0
4. Ceresin wax: 2.0
5. Dextrin palmitate: 2.0
6. Trimethylsiloxysilicic acid: 3.0
7. Dimethyldistearylammonium hectorite: 5.0
8. Propioncarbonate: 1.0
9. Light liquid isoparaffin: balance
10. Copolymer-soluble matter of Synthesis Example 6: 2.0
11. Black iron oxide: 5.0
12. Silica: 3.0
13. Talc: 5.0

### (Manufacturing method)

(1) Components 1 to 5 were heated to 110°C;
(2) Components 6 to 10 were added to and mixed with the components of (1);
(3) Components 11 to 13 were added to and mixed with the mixture of (2); and
(4) The mixture of (3) was treated with a roller to prepare a nonaqueous mascara.

The oil mascara of Example 24 was excellent in "affinity to eyelashes", "makeup-lasting effect", and "absence of sense of burden on eyelashes".

### Example 25: Oil eyeliner

### (Component): (%)

1. Ceresin wax: 11.0
2. Polyisobutylene: 16.0
3. Polyethylene wax: 8.0
4. Light liquid isoparaffin: balance
5. Copolymer-soluble matter of Synthesis Example 7: 2.0
6. Silicone-treated black iron oxide: 15.0
7. Silicone-treated talc: 5.0
8. Preservative: q.s.
9. Perfume: q.s.

### (Manufacturing method)

(1) Components 1 to 5 were heated to 100°C and uniformly mixed;
(2) Components 6 to 9 were heated to 80°C and uniformly mixed;
(3) The mixture of (2) was added to and uniformly mixed with the mixture of (1); and
(4) The mixture of (3) was treated with a roller to prepare an oil eyeliner.

The oil eyeliner of Example 25 was excellent in "affinity to skin", "makeup-lasting effect", and "absence of sense of burden on skin".

Examples using powders coated with methacryloyloxyethyl phosphorylcholine-silicone methacrylate copolymers produced in Manufacturing Examples 1 to 11 will now be described. In each Example, for example, the description "Manufacturing Example 1 (2.0%) treated talc" means "talc coated with 2.0 mass% of the methacryloyloxyethyl phosphorylcholine-silicone methacrylate copolymer described in Manufacturing Example 1", and the description "Manufacturing Example 2 (5.0%) treated titanium oxide" means "titanium oxide coated with 5.0 mass% of the methacryloyloxyethyl phosphorylcholine-silicone methacrylate copolymer described in Manufacturing Example 2". Incidentally, these descriptions do not limit the present invention in any way.

Examples of manufacturing of cosmetics using the copolymers that are used in surface treatment of the present invention will now be described.

### Example 26: O/W type liquid foundation

### (Component): (%)

1. Polyoxyethylene sorbitan monooleate (20 E.O.): 0.5
2. Sorbitan sesquioleate: 0.5
3. 1,3-Butylene glycol: 10.0
4. Manufacturing Example 1 (2.0%) treated titanium oxide: 10.0
5. Manufacturing Example 1 (2.0%) treated red iron oxide: 0.4
6. Manufacturing Example 1 (2.0%) treated yellow iron oxide: 2.0
7. Manufacturing Example 1 (2.0%) treated black iron oxide: 0.1
8. Manufacturing Example 1 (2.0%) treated talc: 5.0
9. Carboxyvinyl polymer: 0.3
10. Triethanolamine: 1.0
11. Purified water: balance
12. Ethanol: 2.0
13. Stearic acid: 1.0
14. Behenyl alcohol: 0.5
15. Liquid paraffin: 2.0
16. Glyceryl tri(2-ethylhexanoate): 2.0
17. 2-Ethylhexyl paramethoxycinnamate: 2.0
18. Vaseline: 0.5
19. Preservative: q.s.
20. Perfume: q.s.

### (Manufacturing method)

(1) Components 1 to 8 were uniformly dispersed with a roller;
(2) Components 9 to 12 were uniformly mixed;
(3) The dispersion of (1) was added to and uniformly mixed with the mixture of (2);
(4) Components 13 to 19 were mixed and dissolved at 80°C;
(5) The mixture of (4) was added to the mixture of (3) at 80°C, followed by emulsification; and
(6) The emulsion of (5) was cooled, and component 20 was added thereto to prepare an O/W type foundation.

The O/W type foundation of Example 26 was excellent in cosmetic film uniformity, makeup-lasting effect, and smooth sense of use.

### [Example 27]

### Oil solid foundation

### (Component): (%)

1. Talc: 15.0
2. Mica: 10.0
3. Manufacturing Example 2 (5.0%) treated titanium oxide: 15.0
4. Manufacturing Example 2 (5.0%) treated red iron oxide: 1.0
5. Manufacturing Example 2 (5.0%) treated yellow iron oxide: 3.0
6. Manufacturing Example 2 (5.0%) treated black iron oxide: 0.2
7. Polyethylene wax: 7.0
8. Microcrystalline wax: 6.0
9. Glyceryl tri(2-ethylhexanoate): balance
10. Dimethylpolysiloxane: 10.0
11. Liquid paraffin: 20.0
12. Polyoxyethylenemethylsiloxane-polyoxypropyleneoleylmethylsiloxane-dimethylsiloxane copolymer (Note 1): 2.0
13. Preservative: q.s.
14. Perfume: q.s.

(Note 1) KF-6026 (manufactured by Shin-Etsu Chemical Co., Ltd.)

### (Manufacturing method)

(1) Components 7 to 13 were heated and dissolved at 90°C;
(2) Components 1 to 6 were added to the solution of (1) and were uniformly dispersed with a roller; and
(3) Component 14 was added to the dispersion of (2) and dissolved at 80°C, and the solution was then put in a metal dish to prepare an oil solid foundation.

The oil solid foundation of Example 27 was excellent in cosmetic film uniformity, makeup-lasting effect, and smooth sense of use.

### [Example 28]

### Stick oil solid concealer

### (Component): (%)

1. Paraffin wax: 5.0
2. Polyethylene wax: 5.0
3. Candelilla wax: 2.0
4. Glyceryl tri(2-ethylhexanoate): balance
5. Methylphenylpolysiloxane: 25.0
6. Liquid lanolin acetate: 10.0
7. 2-Ethylhexyl paramethoxycinnamate: 5.0
8. Manufacturing Example 3 (7.0%) treated titanium oxide: 20.0
9. Manufacturing Example 3 (7.0%) treated yellow iron oxide: 2.0
10. Manufacturing Example 3 (7.0%) treated red iron oxide: 0.5
11. Manufacturing Example 3 (7.0%) treated black iron oxide: 0.2
12. Mica: 7.0
13. Preservative: q.s.

### (Manufacturing method)

(1) Components 1 to 7 were mixed and dissolved at 100°C;
(2) Components 8 to 13 were uniformly mixed with the mixture of (1) at 90°C;
(3) The mixture of (2) was treated with a three-roller mill; and
(4) The mixture of (3) was defoamed and was dissolved and put in a capsule at 85°C and was then cooled at 4°C to prepare a stick concealer.

The stick oil solid concealer of Example 28 was excellent in cosmetic film uniformity, makeup-lasting effect, and smooth sense of use.

### [Example 29]

### Solid powder foundation

### (Component): (%)

1. Manufacturing Example 4 (1.0%) treated talc: 30.0
2. Manufacturing Example 4 (1.0%) treated mica: 15.0
3. Manufacturing Example 4 (1.0%) treated mica titanium oxide: 15.0
4. Manufacturing Example 4 (1.0%) treated sericite: balance
5. Manufacturing Example 4 (1.0%) yellow iron oxide: 2.0
6. Manufacturing Example 4 (1.0%) red iron oxide: 0.5
7. Manufacturing Example 4 (1.0%) black iron oxide: 0.2
8. Synthetic phlogopite: 5.0
9. Crosslinked silicone-networked silicone block copolymer: 1.0
10. Preservative: q.s.
11. Liquid paraffin: 3.0
12. Dimethylpolysiloxane: 3.0
13. Glyceryl 2-ethylhexanoate: 3.0
14. Perfume: q.s.

### (Manufacturing method)

(1) Components 1 to 10 were uniformly dispersed at 75°C with a Henschel mixer (manufactured by Mitsui Miike Machinery Co., Ltd.);
(2) Components 11 to 13 were uniformly mixed and dissolved;
(3) The mixture of (2) and component 14 were added to the dispersion of (1) while stirring with the Henschel mixer to uniformly disperse them;
(4) The dispersion of (3) was pulverized with a pulverizer; and
(5) The powder of (4) was put in a metal dish and pressure-molded to prepare a solid powder foundation.

The solid powder foundation of Example 29 was excellent in cosmetic film uniformity, makeup-lasting effect, and smooth sense of use.

### [Example 30]

### Solid powder eye shadow

### (Component): (%)

1. Manufacturing Example 5 (1.0%) treated synthetic gold mica: 10.0
2. Manufacturing Example 5 (1.0%) treated talc: balance
3. Titanium oxide-coated mica: 30.0
4. Boron nitride: 5.0
5. Polyethylene terephthalate-aluminum-epoxy laminated powder: 5.0
6. Ultramarine blue: 2.0
7. Red No. 202: 0.5
8. Alkyl polyacrylate: 1.0
9. Preservative: q.s.
10. Liquid paraffin: 3.0
11. Dimethylpolysiloxane: 5.0
12. Glyceryl 2-ethylhexanoate: 3.0
13. Perfume: q.s.

### (Manufacturing method)

(1) Components 1 to 9 were uniformly dispersed at 75°C with a Henschel mixer (manufactured by Mitsui Miike Machinery Co., Ltd.);
(2) Components 10 to 12 were uniformly mixed and dissolved;
(3) The mixture of (2) and component 13 were added to the dispersion of (1) while stirring with the Henschel mixer to uniformly disperse them;
(4) The dispersion of (3) was pulverized with a pulverizer; and
(5) The powder of (4) was put in a metal dish and pressure-molded to prepare a solid powder eye shadow.

The solid powder eye shadow of Example 30 was excellent in cosmetic film uniformity, makeup-lasting effect, and smooth sense of use.

### [Example 31]

### Solid powder face color

### (Component): (%)

1. Manufacturing Example 6 (1.0%) treated mica: 20.0
2. Manufacturing Example 6 (1.0%) treated talc: balance
3. Titanium oxide-coated mica: 10.0
4. Ultramarine blue: 0.5
5. Red No. 226: 0.2
6. Alkyl polyacrylate: 1.0
7. Preservative: q.s.
8. Liquid paraffin: 2.0
9. Dimethylpolysiloxane: 3.0
10. Glyceryl 2-ethylhexanoate: 3.0
11. Perfume: q.s.

### (Manufacturing method)

(1) Components 1 to 7 were uniformly dispersed at 75°C with a Henschel mixer (manufactured by Mitsui Miike Machinery Co., Ltd.);
(2) Components 8 to 10 were heated to 65°C and uniformly mixed and dissolved;
(3) The mixture of (2) and component 11 were added to the dispersion of (1) while stirring with the Henschel mixer to uniformly disperse them;
(4) The dispersion of (3) was pulverized with a pulverizer; and
(5) The powder of (4) was put in a metal dish and pressure-molded to prepare a solid powder face color.

The solid powder face color of Example 31 was excellent in cosmetic film uniformity, makeup-lasting effect, and smooth sense of use.

### [Example 32]

### Powdery face powder

### (Component): (%)

1. Manufacturing Example 7 (0.5%) treated mica: 20.0
2. Manufacturing Example 7 (0.5%) treated talc: balance
3. Titanated mica: 10.0
4. Red No. 226: 0.5
5. Liquid paraffin: 0.5
6. Glyceryl tri(2-ethylhexanoate): 1.0
7. Preservative: q.s.
8. Perfume: q.s.

### (Manufacturing method)

(1) Components 1 to 4 were uniformly mixed;
(2) Component 5 to 8 were added to the mixture of (1) while stirring with a Henschel mixer to uniformly mix them; and
(3) The mixture of (2) was pulverized with a pulverizer to prepare a face powder.

The powdery face powder of Example 32 was excellent in cosmetic film uniformity, makeup-lasting effect, and smooth sense of use.

### [Example 33]

### Stick rouge

### (Component): (%)

1. Polyethylene wax: 10.0
2. Carnauba wax: 5.0
3. Paraffin wax: 2.0
4. Cetyl 2-ethylhexanoate: balance
5. Liquid paraffin: 10.0
6. Isotridecyl isononanate: 10.0
7. Dimethylpolysiloxane: 5.0
8. Red No. 202: 0.5
9. Yellow No. 4: 2.0
10. Manufacturing Example 8 (2.0%) treated titanated mica: 3.0
11. Manufacturing Example 8 (2.0%) treated titanium oxide: 0.5
12. Manufacturing Example 8 (2.0%) treated black iron oxide: 0.1
13. α-Tocopherol: 0.5
14. Perfume: q.s.

### (Manufacturing method)

(1) Components 1 to 7 were uniformly dissolved and mixed at 100°C;
(2) Components 8 to 14 were added to and uniformly mixed with the mixture of (1); and
(3) The mixture of (2) was flown into a container and cooled to prepare a stick rouge.

The stick rouge of Example 33 was excellent in cosmetic film uniformity, makeup-lasting effect, and smooth sense of use.

### [Example 34]

### O/W type mascara

### (Component): (%)

1. Stearic acid: 2.0
2. Bees wax: 10.0
3. Cetostearyl alcohol: 1.0
4. Polyoxyethylene sorbitan monooleate (20 E.O.): 1.5
5. Sorbitan sesquioleate: 0.5
6. Dimethylpolysiloxane: 5.0
7. Manufacturing Example 9 (5.0%) treated black iron oxide: 5.0
8. Silicic anhydride: 3.0
9. Purified water: balance
10. 1,3-Butylene glycol: 10.0
11. Triethanolamine: 1.5
12. Alkyl acrylate copolymer emulsion (Note 1): 30.0
13. Preservative: q.s.
14. Perfume: q.s.

(Note 1) Yodosol 32A707 (solid content: 45%) (manufactured by Nippon NSC Ltd.)

### (Manufacturing method)

(1) Components 1 to 3 were uniformly mixed at 80°C;
(2) Components 4 to 8 were treated with a roller;
(3) Components 9 to 14 were uniformly mixed at 80°C;
(4) The mixture of (1) and the components of (2) were mixed, and the mixture of (3) was added thereto, followed by emulsification; and
(5) The emulsion of (4) was cooled to prepare an O/W type mascara.

The O/W type mascara of Example 34 was excellent in cosmetic film uniformity, makeup-lasting effect, and smooth sense of use.

### [Example 35]

### Oil mascara

### (Component): (%)

1. Pentaerythrityl rosinate: 10.0
2. Candekilla resin: 3.0
3. Bees wax: 2.0
4. Ceresin wax: 2.0
5. Dextrin palmitate: 2.0
6. Trimethylsiloxysilicic acid: 3.0
7. Dimethyldistearylammonium hectorite: 5.0
8. Propioncarbonate: 1.0
9. Light liquid isoparaffin: balance
10. Dimethylpolysiloxane: 3.0
11. Manufacturing Example 1 (10%) treated black iron oxide: 5.0
12. Silica: 3.0
13. Talc: 5.0

### (Manufacturing method)

(1) Components 1 to 5 were heated to 110°C;
(2) Components 6 to 10 were added to and mixed with the components of (1);
(3) Components 11 to 13 were added to and mixed with the mixture of (2); and
(4) The mixture of (3) was treated with a three-roller mill to prepare an oil mascara.

The oil mascara of Example 35 was a nonaqueous mascara being excellent in cosmetic film uniformity, makeup-lasting effect, and smooth sense of use.

### [Example 36]

### Oil eyeliner

### (Component): (%)

1. Ceresin wax: 11.0
2. Polyisobutylene: 16.0
3. Polyethylene wax: 8.0
4. Light liquid isoparaffin: balance
5. Dimethylpolysiloxane: 3.0
6. Manufacturing Example 2 (0.5%) treated black iron oxide: 15.0
7. Manufacturing Example 2 (0.5%) treated talc: 5.0
8. Preservative: q.s.
9. Perfume: q.s.

### (Manufacturing method)

(1) Components 1 to 5 were heated to 100°C and uniformly mixed;
(2) Components 6 to 9 were heated to 80°C and uniformly mixed;
(3) The mixture of (2) was added to and uniformly mixed with the mixture of (1); and
(4) The mixture of (3) was treated with a roller to prepare an oil eyeliner.

The oil eyeliner of Example 36 was excellent in cosmetic film uniformity, makeup-lasting effect, and smooth sense of use.

### [Example 37]

### W/O type sunscreen

### (Component): (%)

1. Manufacturing Example 1 (5.0%) treated zinc oxide: 2.0
2. Manufacturing Example 1 (5.0%) treated fine titanium oxide: 5.0
3. Caprylic/capric triglyceride: 5.0
4. Dimethylpolysiloxane: 3.0
5. Octyl palmitate: 3.0
6. 2-Ethylhexyl paramethoxycinnamate: 10.0
7. Decamethylcyclopentasiloxane: 10.0
8. Methylpolysiloxane-cetylmethylpolysiloxane-poly(oxyethylene/oxypropylene)methylpolysiloxane copolymer (Note 1): 1.8
9. Glyceryl tri(2-ethylhexanoate): 3.0
10. Preservative: q.s.
11. Sodium chloride: 0.3
12. Purified water: balance
13. Dipropylene glycol: 3.0
14. Ethanol: 3.0
15. Perfume: q.s.

(Note 1) ABIL EM-90 (manufactured by Evonik Goldschmidt GmbH)

### (Manufacturing method)

(1) Components 3 and 4 were heated and dissolved, and components 1 and 2 were added thereto, followed by uniform dispersion with a three-roller mill;
(2) Components 5 to 10 were dissolved at 70°C, and the dispersion of (1) was added thereto at 60°C, followed by uniform mixing and dissolving;
(3) Components 11 to 13 were mixed and dissolved and were then added to the mixture of (2) at 60°C, followed by emulsification; and
(4) Components 14 and 15 were added to and uniformly mixed with the emulsion of (3) to prepare a W/O type sunscreen.

The W/O type sunscreen of Example 37 was excellent in cosmetic film uniformity, makeup-lasting effect, and smooth sense of use.

### Industrial Applicability

The copolymer of the present invention is not only significantly excellent in solubility in a low polar oil and adhesion to a powder and also excellent in bioaffinity, moisture retention, water repellency, water resistance, and oil resistance. Accordingly, cosmetics manufactured using the copolymer of the present invention or the surface-treated powder of the present invention have skin affinity, moisture retention, water repellency, lubricity, and touch-proof properties of not easily coming off even if touched. Accordingly, the present invention contributes to improve, in particular, the cosmetic manufacturing technology and is a significantly useful invention.

## Claims

1. A copolymer comprising the following (i) to (iv) as repeating units:
(i) methacryloyloxyethyl phosphorylcholine represented by the following formula or a derivative thereof: 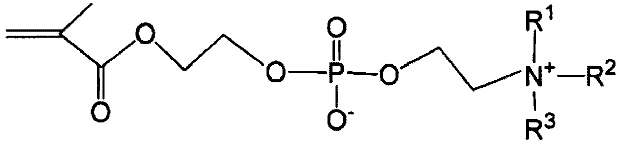 wherein R¹, R², and R³ are the same as or different from each other and each represent a substituted or unsubstituted linear or branched alkyl group having 1 to 30 carbon atoms ;
(ii) a silicone methacrylate represented by the following formula: 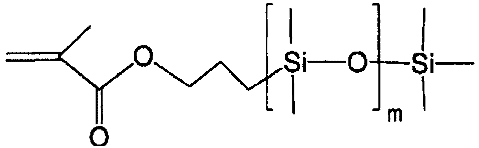 wherein m represents an integer of 1 to 30;
(iii) a methacrylate represented by the following formula: 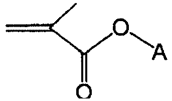 wherein A is selected from the group consisting of a substituted or unsubstituted linear or branched alkyl group having 1 to 30 carbon atoms, a substituted or unsubstituted linear or branched alkenyl group having 2 to 30 carbon atoms, and a substituted or unsubstituted cycloalkyl group having 3 to 20 carbon atoms; and
(iv) a methoxypolyethylene glycol methacrylate represented by the following formula: 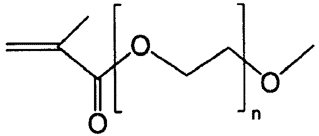 wherein n represents an integer of 2 to 30.

2. The copolymer according to claim 1, wherein R¹, R², and R³ in (i) are the same as or different from each other and each represent a substituted or unsubstituted linear or branched alkyl group having 1 to 3 carbon atoms.

3. The copolymer according to claim 1 or 2, wherein m in (ii) is 8 to 16.

4. The copolymer according to any one of claims 1 to 3, wherein A in (iii) is a substituted or unsubstituted linear or branched alkyl group having 1 to 30 carbon atoms or a substituted or unsubstituted cycloalkyl group having 3 to 8 carbon atoms.

5. The copolymer according to any one of claims 1 to 4, wherein n in (iv) is 2 to 25.

6. The copolymer according to any one of claims 1 to 5, wherein the copolymer contains (i) in an amount of 0.5 to 10 mass% based on the copolymer mass,
wherein the copolymer contains (ii) in an amount of 0.5 to 10 mass% based on the copolymer mass,
wherein the copolymer contains (iii) in an amount of 75 to 90 mass% based on the copolymer mass, and
wherein the copolymer contains (iv) in an amount of 1 to 5 mass% based on the copolymer mass.

7. The copolymer according to any one of claims 1 to 6, wherein the copolymer has a weight-average molecular weight of 10,000 to 150,000, calculated by a multi-angle light scattering method (MALS) using a multi-angle light scattering detector.

8. An oil dispersion comprising the copolymer according to any one of claims 1 to 7 and an oil agent.

9. A cosmetic comprising the copolymer according to any one of claims 1 to 7.

10. A cosmetic comprising the oil dispersion according to claim 8.

11. A surface-treated powder coated with the copolymer according to any one of claims 1 to 7.

12. The surface-treated powder according to claim 11, wherein a coating weight of the copolymer is 0.1 to 5.0 mass% of the total amount of the copolymer and the powder.

13. The surface-treated powder according to claim 11 or 12, wherein the powder is one or more powders selected from minerals and metal oxides.

14. A cosmetic comprising the surface-treated powder according to any one of claims 11 to 13.

15. A method for manufacturing the surface-treated powder according to any one of claims 11 to 13, comprising the following steps (a) to (d):
(a) dissolving the copolymer according to claim 1 in a solvent with a stirring kneading machine;
(b) adding a powder to the resulting solution and kneading a mixture of the powder and the solution in a form of slurry;
(c) kneading the mixture while removing the solvent in vacuum and taking out the resulting powder; and
(d) drying and then pulverizing the taken-out powder.

## Patentansprüche

1. Copolymer, das die folgenden Einheiten (i) bis (iv) als sich wiederholende Einheiten umfasst:
(i) Methacryloyloxyethylphosphorylcholin, dargestellt durch die folgende Formel oder ein Derivat davon: 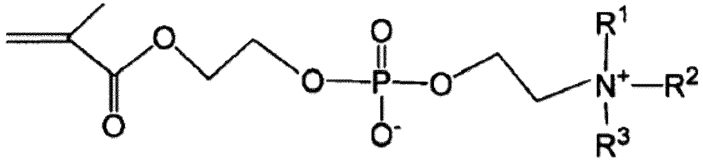 wobei
R¹, R² und R³ gleich oder verschieden voneinander sind und jeweils eine substituierte oder unsubstituierte lineare oder verzweigte Alkylgruppe mit 1 bis 30 Kohlenstoffatomen darstellen ;
(ii) ein Siliconmethacrylat, dargestellt durch die folgende Formel: 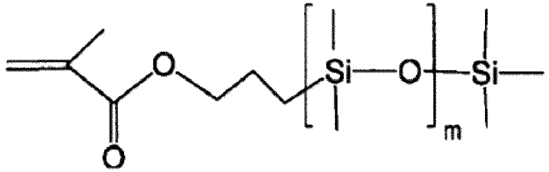 ist, wobei m eine ganze Zahl von 1 bis 30 darstellt;
(iii) ein Methacrylat, dargestellt durch die folgende Formel: 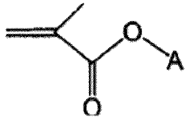 ist, wobei
A aus der Gruppe ausgewählt ist, die aus einer substituierten oder unsubstituierten linearen oder verzweigten Alkylgruppe mit 1 bis 30 Kohlenstoffatomen, einer substituierten oder unsubstituierten linearen oder verzweigten Alkenylgruppe mit 2 bis 30 Kohlenstoffatomen und einer substituierten oder unsubstituierten Cycloalkylgruppe mit 3 bis 20 Kohlenstoffatomen besteht; und
(iv) ein Methoxypolyethylenglykolmethacrylat, dargestellt durch die folgende Formel: 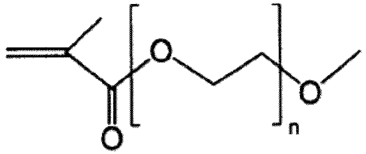 ist, wobei n eine ganze Zahl von 2 bis 30 darstellt.

2. Copolymer nach Anspruch 1, wobei R¹, R² und R³ in (i) gleich oder verschieden voneinander sind und jeweils eine substituierte oder unsubstituierte lineare oder verzweigte Alkylgruppe mit 1 bis 3 Kohlenstoffatomen darstellen.

3. Copolymer nach Anspruch 1 oder 2, wobei m in (ii) 8 bis 16 ist.

4. Copolymer nach einem der Ansprüche 1 bis 3, wobei A in (iii) eine substituierte oder unsubstituierte lineare oder verzweigte Alkylgruppe mit 1 bis 30 Kohlenstoffatomen oder eine substituierte oder unsubstituierte Cycloalkylgruppe mit 3 bis 8 Kohlenstoffatomen ist.

5. Copolymer nach einem der Ansprüche 1 bis 4, wobei n in (iv) 2 bis 25 ist.

6. Copolymer nach einem der Ansprüche 1 bis 5, wobei das Copolymer (i) in einer Menge von 0,5 bis 10 Massen-%, bezogen auf die Copolymermasse, enthält,
wobei das Copolymer (ii) in einer Menge von 0,5 bis 10 Massen-%, bezogen auf die Copolymermasse, enthält,
wobei das Copolymer (iii) in einer Menge von 75 bis 90 Massen-%, bezogen auf die Copolymermasse, enthält, und
wobei das Copolymer (iv) in einer Menge von 1 bis 5 Massen-%, bezogen auf die Copolymermasse, enthält.

7. Copolymer nach einem der Ansprüche 1 bis 6, wobei das Copolymer ein gewichtsmittleres Molekulargewicht von 10.000 bis 150.000 aufweist, berechnet durch eine Mehrwinkel-Lichtstreuungsmethode (MALS) unter Verwendung eines Mehrwinkel-Lichtstreuungsdetektors.

8. Öldispersion, umfassend das Copolymer nach einem der Ansprüche 1 bis 7 und ein Ölmittel.

9. Kosmetikum, umfassend das Copolymer nach einem der Ansprüche 1 bis 7.

10. Kosmetikum, das die Öldispersion nach Anspruch 8 umfasst.

11. Oberflächenbehandeltes Pulver, das mit dem Copolymer nach einem der Ansprüche 1 bis 7 beschichtet ist.

12. Oberflächenbehandeltes Pulver nach Anspruch 11, wobei das Beschichtungsgewicht des Copolymers 0,1 bis 5,0 Massen-% der Gesamtmenge des Copolymers und des Pulvers beträgt.

13. Oberflächenbehandeltes Pulver nach Anspruch 11 oder 12, wobei das Pulver ein oder mehrere Pulver ist, die aus Mineralien und Metalloxiden ausgewählt sind.

14. Kosmetikum, das das oberflächenbehandelte Pulver nach einem der Ansprüche 11 bis 13 umfasst.

15. Verfahren zum Herstellen des oberflächenbehandelten Pulvers nach einem der Ansprüche 11 bis 13, umfassend die folgenden Schritte (a) bis (d):
(a) Lösen des Copolymers nach Anspruch 1 in einem Lösungsmittel mit einer Rührknetmaschine;
(b) Zugabe eines Pulvers zu der resultierenden Lösung und Kneten einer Mischung aus dem Pulver und dem Lösen in Form eines Schlamms;
(c) Kneten der Mischung unter Entfernen des Lösungsmittels im Vakuum und Entnahme des entstandenen Pulvers; und
(d) Trocknen und anschließendes Pulverisieren des entnommenen Pulvers.

## Revendications

1. Copolymère comprenant les composés suivants (i) à (iv) en tant que motifs répétitifs :
(i) méthacryloyloxyéthyle phosphorylcholine représentée par la formule suivante ou un dérivé correspondant : 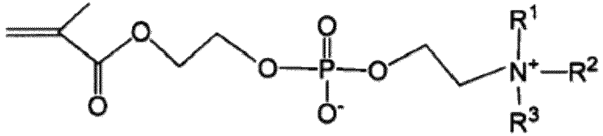 R¹, R² et R³ étant identiques ou différents les uns des autres et représentant chacun un groupe alkyle substitué ou non substitué linéaire ou ramifié possédant 1 à 30 atomes de carbone ;
(ii) un méthacrylate de silicone représenté par la formule suivante : 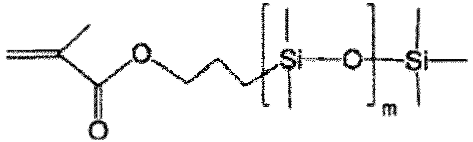 m représentant un entier de 1 à 30 ;
(iii) un méthacrylate représenté par la formule suivante : 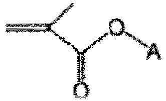 A étant choisi dans le groupe constitué par un groupe alkyle substitué ou non substitué linéaire ou ramifié possédant 1 à 30 atomes de carbone, un groupe alcényle substitué ou non substitué linéaire ou ramifié possédant 2 à 30 atomes de carbone et un groupe cycloalkyle substitué ou non substitué possédant 3 à 20 atomes de carbone ; et
(iv) un méthacrylate de méthoxy-poly(éthylène glycol) représenté par la formule suivante : 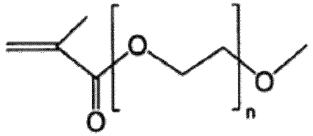 n représentant un entier de 2 à 30.

2. Copolymère selon la revendication 1, R¹, R² et R³ en (i) étant identiques ou différents les uns des autres et représentant chacun un groupe alkyle substitué ou non substitué linéaire ou ramifié possédant 1 à 3 atomes de carbone.

3. Copolymère selon la revendication 1 ou 2, m en (ii) étant 8 à 16.

4. Copolymère selon l'une quelconque des revendications 1 à 3, A en (iii) étant un groupe alkyle substitué ou non substitué linéaire ou ramifié possédant 1 à 30 atomes de carbone ou un groupe cycloalkyle substitué ou non substitué possédant 3 à 8 atomes de carbone.

5. Copolymère selon l'une quelconque des revendications 1 à 4, n en (iv) étant 2 à 25.

6. Copolymère selon l'une quelconque des revendications 1 à 5,
le copolymère contenant (i) en une quantité de 0,5 à 10 % en masse sur la base de la masse du copolymère,
le copolymère contenant (ii) en une quantité de 0,5 à 10 % en masse sur la base de la masse du copolymère,
le copolymère contenant (iii) en une quantité de 75 à 90 % en masse sur la base de la masse du copolymère, et
le copolymère contenant (iv) en une quantité de 1 à 5 % en masse sur la base de la masse du copolymère.

7. Copolymère selon l'une quelconque des revendications 1 à 6, le copolymère possédant un poids moléculaire moyen en poids de 10 000 à 150 000, calculé par un procédé de diffusion de lumière multi-angle (MALS) en utilisant un détecteur de diffusion de lumière multi-angle.

8. Dispersion d'huile comprenant le copolymère selon l'une quelconque des revendications 1 à 7 et un agent de type huile.

9. Cosmétique comprenant le copolymère selon l'une quelconque des revendications 1 à 7.

10. Cosmétique comprenant la dispersion d'huile selon la revendication 8.

11. Poudre traitée en surface revêtue par le copolymère selon l'une quelconque des revendications 1 à 7.

12. Poudre traitée en surface selon la revendication 11, un poids de revêtement du copolymère étant de 0,1 à 5,0 % en masse de la quantité totale du copolymère et de la poudre.

13. Poudre traitée en surface selon la revendication 11 ou 12, la poudre étant une ou plusieurs poudres choisies parmi des minéraux et des oxydes métalliques.

14. Cosmétique comprenant la poudre traitée en surface selon l'une quelconque des revendications 11 à 13.

15. Procédé pour la fabrication de la poudre traitée en surface selon l'une quelconque des revendications 11 à 13, comprenant les étapes suivantes (a) à (d) :
(a) dissolution du copolymère selon la revendication 1 dans un solvant avec un malaxeur à agitation ;
(b) ajout d'une poudre à la solution résultante et malaxage d'un mélange de la poudre et de la solution sous une forme de bouillie ;
(c) malaxage du mélange tout en éliminant le solvant sous vide et retrait de la poudre résultante ;
(d) séchage et ensuite pulvérisation de la poudre retirée.
